# FASCICULE DE BREVET EUROPEEN

(11) **EP 0 573 360 B1**
(45) Date de publication et mention de la délivrance du brevet: **26.08.1998**
(21) Numéro de dépôt: 93401416.8
(22) Date de dépôt: 03.06.1993
(51) Int. Cl.: C07D 495/14, A61K 31/495

(54) **Composés pyrrolothiénopyraziniques comme antagonistes des récepteurs 5-HT3**
Pyrrolothienopyrazin-Verbindungen als 5HT3-Rezeptoren-Antagonisten
Pyrrolothienopyrazine compounds as 5-HT3 receptors antagonists

(30) Priorité: 05.06.1992 FR 9206800
(43) Date de publication de la demande: 08.12.1993
(73) Titulaire: ADIR ET COMPAGNIE, 92415 Courbevoie Cédex (FR)
(72) Inventeur: Rault, Sylvain, F-14370 Moult (FR); Lancelot, Jean-Charles, F-14400 Calvados (FR); Pilo Vincente, Juan-Carlos, E-49220 Fermoselle (Zamora) (ES); Robba, Max, F-75004 Paris (FR); Guardiola-Lemaitre, Béatrice, f-92200 Neuilly sur Seine (FR); Renard, Pierre, F-78000 Versailles (FR); Adam, Gérard, F-78600 Le Mesnil le Roi (FR)

(56) Documents cités:
- EP-A- 0 446 140
- EP-A- 0 449 728
- JOURNAL OF HETEROCYCLIC CHEMISTRY. vol. 24, no. 2, Mars 1987, PROVO US pages 431 - 435 Y. EFFI ET AL. 'Pyrrolothiénopyrazines. IV. Synthèse de dérivés de l'amino-5 pyrrolo(1,2-a)thiéno(2,3-e)pyrazine'
- JOURNAL OF HETEROCYCLIC CHEMISTRY. vol. 20, no. 4, Juillet 1983, PROVO US PAGES 913 - 918 Y. EFFI ET AL. 'Pyrrolo(1,2-a)thiéno(2,3-e)pyrazine. Etude chimique de la dihydro-4,5 oxo-5 pyrrolo(1,2-a)thiéno(2,3-e)pyrazine'
- JOURNAL OF HETEROCYCLIC CHEMISTRY. vol. 18, no. 4, Juin 1981, PROVO US pages 739 - 742 S. RAULT EZ AL. 'Pyrrolo(1,2-a)thiéno(3,2-e)pyrazines'
- CHEMICAL ABSTRACTS, vol. 107, no. 7, 17 Août 1987, Columbus, Ohio, US; abstract no. 51815e, R. NEALE ET AL. 'Biochemical and pharmacological characterization of CGS 12066B, a selective serotonin-1B agonist' page 58 ;

## Description

La présente invention concerne de nouveaux composés pyrrolothiénopyraziniques, leurs procédés de préparation et les compositions pharmaceutiques qui les contiennent.

La demanderesse a présentement découvert de nouveaux composés pyrrolothiénopyraziniques qui montrent une très forte affinité pour certains récepteurs sérotoninergiques. Ils montrent notamment une remarquable affinité pour les récepteurs 5-HT₃ sélective vis à vis des autres récepteurs sérotoninergiques.

On connait, dans la littérature, la 4-(4-méthylpipérazinyl)-7-(trifluorométhyl)pyrrolo[1,2-a]quinoxaline, qui est décrite uniquement comme un agoniste des récepteurs sérotoninergiques 5HT-1B (Neale R.F. et al, Eur. J. of Pharmacology, 1987, 136, pp 1-9 et Macor J. E. et al J. Med. Chem. 1990, 33, pp 2087-2093).

Plus particulièrement, la présente invention concerne les composés de formule (I) : 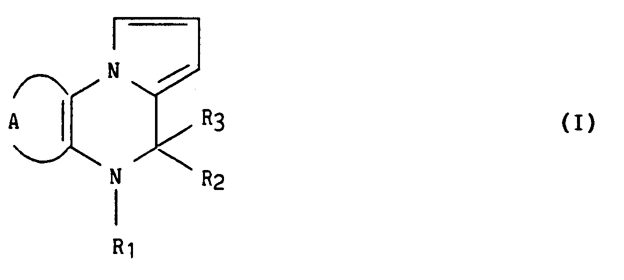 dans laquelle :
- soit R₁ et R₂ forment ensemble une double liaison et R₃ représente un atome de chlore ou un groupement de formule : 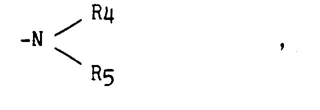 dans laquelle R₄ et R₅ forment avec l'atome d'azote qui les porte un groupement choisi parmi :
   - pipérazine,
   - pipérazine substituée,
   - pipéridine substituée,
   - pyrrolidine substituée,
   - morpholine substituée par un ou plusieurs radicaux alkyles,
   - tétrahydropyridine,
   - thiomorpholine,
   - azaspirane de 5 à 12 chaînons,
   - azaspirane de 5 à 12 chaînons substitué par un ou plusieurs radicaux alkyles ou groupements oxo,
   - azacycloalkyle de 7 à 12 chaînons incluant éventuellement dans son squelette de 1 à 2 hétéroatomes supplémentaires choisis parmi oxygène, soufre, et azote,
   - azacycloalkyle de 7 à 12 chaînons substitué par un ou plusieurs radicaux alkyles ou groupements oxo incluant éventuellement dans son squelette de 1 à 2 hétéroatomes supplémentaires choisis parmi oxygène, soufre, et azote,
   - un groupement -NH-(CH₂)ₖ-NH₂ dans lequel k représente un nombre entier égal à 2, 3, ou 4,
   - et un groupement -NH-(CH₂)ₖ-NH₂ substitué dans lequel k représente un nombre entier égal à 2, 3, ou 4,
- soit R₁ représente un atome d'hydrogène, et R₂ et R₃ forment ensemble un groupement oxo,
   étant entendu que le terme "substitué(e)" affectant ci-dessus les groupements pipérazine, pipéridine, pyrrolidine, et -NH-(CH₂)ₖ-NH₂ signifie que ces groupements peuvent être substitués par un ou plusieurs atomes d'halogène, radicaux hydroxyles, radicaux oxo, radicaux R₁₀, ou radicaux 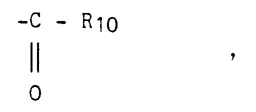 avec R₁₀ étant choisi parmi :
   - alkyle,
   - alkoxy,
   - alcényle,
   - (CH₂)ₙ-R₁₁ ou 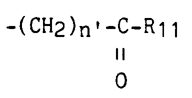 où n représente 0 ou un nombre entier de 1 à 5, n' représente un nombre entier de 1 à 5, et où R₁₁ représente un radical phényle, benzhydryle, thiényle, pyrrolyle, pyrrolydinyle, furyle, pyrimidinyle, pyridyle, benzodioxolyle, benzodioxanyle, naphtyle, quinoléinyle, isoquinoléinyle, cycloalkyle, et dicycloalkylméthyle, le terme cycloalkyle représentant un groupement mono- ou bi-cyclique, de 3 à 12 chaînons, ces radicaux R₁₁ pouvant eux-mêmes être substitués par un ou plusieurs radicaux choisis parmi halogène, trifluorométhyle, amino, nitro, hydroxyle, alkyle, et alkoxy,
   - et -(CH₂)_{n'}-R₁₂ où n' est tel que défini précédemment et R₁₂ représente un groupement choisi parmi carboxyle, alkoxycarbonyle, amino, dialkylamino, -SO₂NR₁₃R₁₄, et -CONR₁₃R₁₄ dans lesquels R₁₃ et R₁₄ représentent chacun, indépendamment l'un de l'autre, un atome d'hydrogène ou un alkyle,

   . et A signifie :
- soit un groupement de formule (α) : 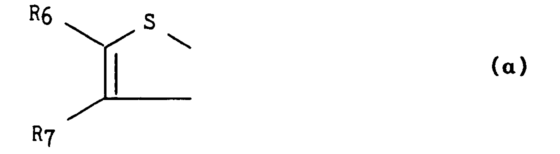 et forme avec le système hétérocyclique qui le porte une pyrrolo[1,2-a] thiéno[3,2-e]pyrazine de formule (I/α) : 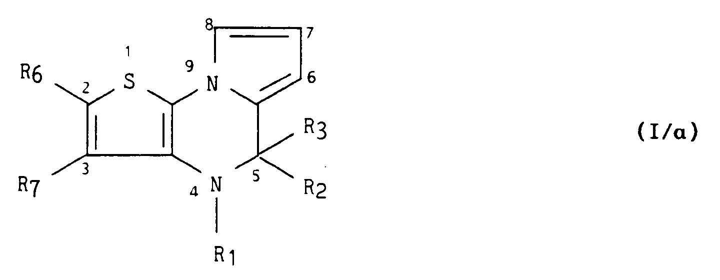 dans laquelle R₁, R₂, et R₃ sont tels que définis précédemment et R₆ et R₇, identiques ou différents, représentent chacun indépendamment l'un de l'autre un groupement choisi parmi :
   - hydrogène,
   - alkyle,
   - alkyle substitué par un ou plusieurs atomes d'halogène ou radicaux hydroxyle,
   - acyle,
   - alkoxycarbonyle,
   - halogène,
   - nitro,
   - -(CH₂)ₘ-phényle, -(CH₂)ₘ-napthyle, ou -(CH₂)ₘ-pyridyle, où m représente 0 ou un nombre entier de 1 à 4 et où le noyau phényle est non substitué ou substitué par un ou plusieurs radicaux Ra, identiques ou différents, avec Ra signifiant halogène, alkyle, alkoxy, trifluorométhyle, ou pyrolidinyle,
   - et un groupe de formule : 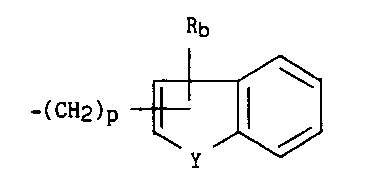 dans laquelle p représente 0 ou un nombre entier de 1 à 2, Y représente un atome d'oxygène, d'azote, ou de soufre et R_{b} représente un atome d'hydrogène ou un groupement alkyle,
- soit un groupement de formule (β) : 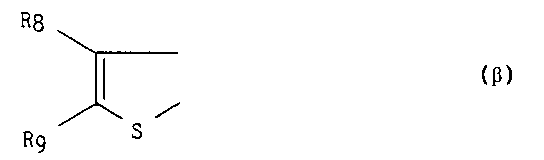 et forme avec le système hétérocyclique qui le porte une pyrrolo [1,2-a] thiéno[2,3-e]pyrazine de formule (I/β) : 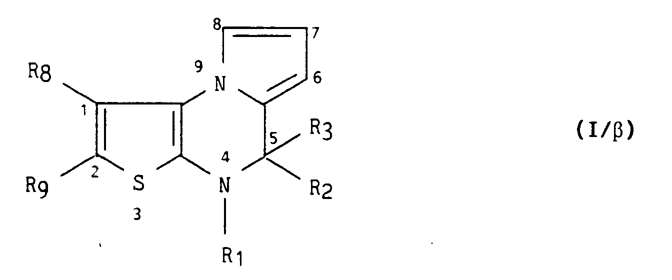 dans laquelle R₁, R₂, et R₃ sont tels que définis précédemment et R₈ et R₉ ont les mêmes significations que R₆ et R₇ tels que définis précédemment,
   étant entendu que si R₃ représente un atome de chlore ou si R₃ et R₂ forment ensemble un groupement oxo, alors R₆ et R₇, ou R₈ et R₉, ne peuvent pas représenter simultanément deux atomes d'hydrogène,
   leurs isomères optiques, sous forme pure ou sous forme de mélange, quand R₃, R₆, R₇, R₈ ou R₉ représente un groupement optiquement actif,
   et leurs sels d'addition à un acide ou à une base pharmaceutiquement acceptable,
   étant entendu que, sauf précisions contraires,
- les termes "alkyle", "alkoxy", et "acyle" représentent des groupements linéaires ou ramifiés possédant de 1 à 6 atomes de carbone,
- et le terme "alcényle" représente un groupement insaturé, linéaire ou ramifié, possédant de 2 à 6 atomes de carbone.

Plus particulièrement, l'invention concerne les composés de formule (I) dans laquelle R₁ et R₂ forment ensemble une double liaison et R₃ représente un groupement de formule : 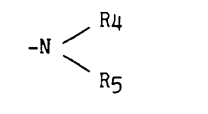 où R₄ et R₅ sont tels que définis dans la formule (I),
et par exemple, les composés de formule (I) dans laquelle R₄ et R₅ forment avec l'atome d'azote qui les porte une pipérazine ou une pipérazine substituée, le terme "substituée" étant tel que défini dans la formule (I),
leurs isomères optiques,
et leurs sels d'addition à un acide ou à une base pharmaceutiquement acceptable.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

La présente invention s'étend au procédé de préparation des composés de formule (I) caractérisé en ce que :
on chauffe, en présence ou en absence de solvant, un composé de formule (II), 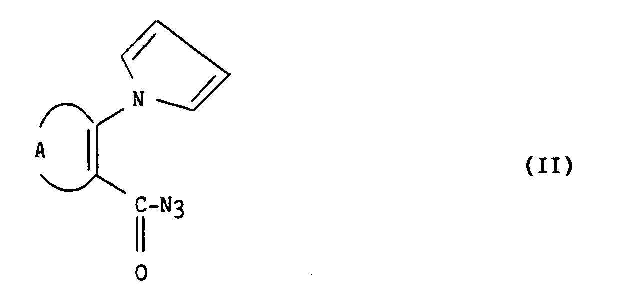 dans laquelle A est tel que défini dans la formule (I),
pour obtenir un composé de formule (I/a) : 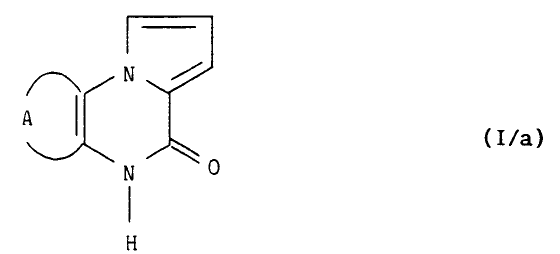 dans laquelle A est tel que défini précédemment,
cas particulier des composés de formule (I) dans lesquels R₁ représente un atome d'hydrogène et R₂ et R₃ forment ensemble un groupement oxo,
composé de formule (I/a) qui est soumis à un agent d'halogénation pour obtenir un composé de formule (I/b) : 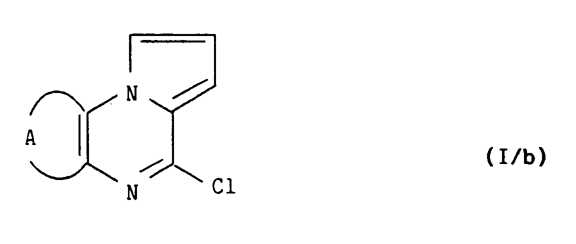 dans laquelle A est tel que défini précédemment,
cas particulier des composés de formule (I) dans lesquels R₁ et R₂ forment ensemble une double liaison et R₃ représente un atome de chlore,
qui est ensuite mis en réaction avec une amine de formule (III) : 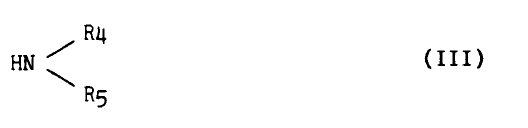 dans laquelle R₄ et R₅ sont tels que définis dans la formule (I),
pour obtenir un composé de formule (I/c) : 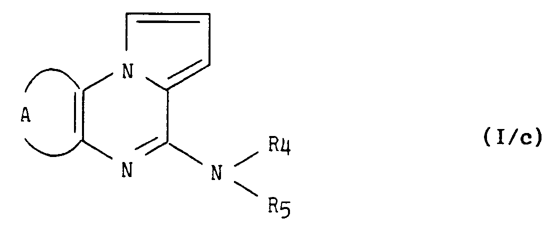 dans laquelle A, R₄, et R₅ sont tels que définis précédemment,
cas particulier des composés de formule (I) dans lesquels R₁ et R₂ forment une double liaison et R₃ représente un groupement : 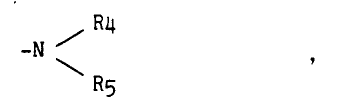 les composés de formule (I/a), (I/b), (I/c) formant l'ensemble des composés de formule (I),
les composés de formule (I) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- et/ou salifiés par un acide ou à une base pharmaceutiquement acceptable.

L'invention s'étend également au procédé de préparation des composés de formule (I/c), cas particulier des composés de formule (I) dans lesquels R₁ et R₂ forment ensemble une double liaison et R₃ représente un groupement : 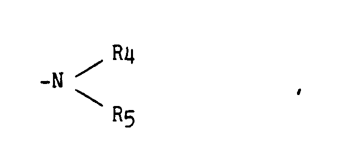 caractérisé en ce que :
on fait réagir un composé de formule (II) : 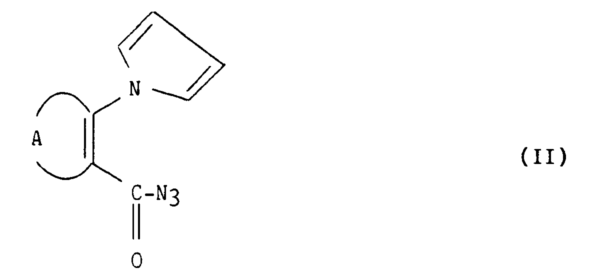 dans laquelle A est tel que défini dans la formule (I),
avec une amine de formule (III) : 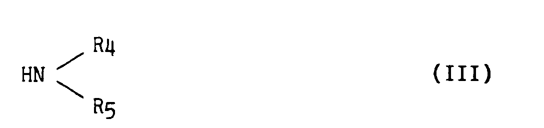 dans laquelle R₄ et R₅ sont tels que définis dans la formule (I) pour obtenir un composé de formule (V) : 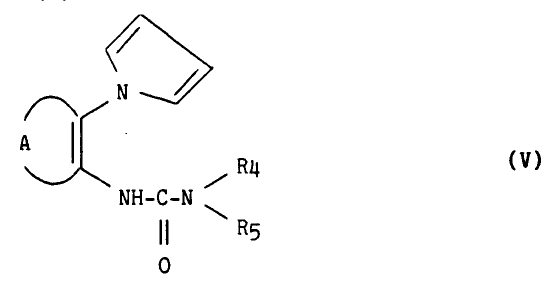 dans laquelle A, R₄ et R₅ sont tels que définis précédemment,
qui est ensuite cyclisé sous l'action d'un agent d'halogénation et soumis à un traitement alcalin pour donner un composé de formule (I/c) : 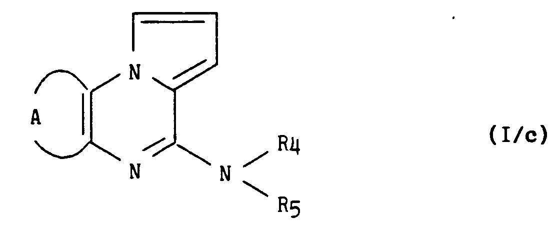 dans laquelle A, R₄, et R₅ sont tels que définis précédemment,
cas particulier des composés de formule (I) dans lesquels R₁ et R₂ forment ensemble une double liaison et R₃ représente un groupement : 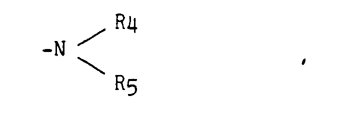 les composés de formule (I/c) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- et/ou salifiés par un acide où à une base pharmaceutiquement acceptable.

Les composés de formule (II) sont aisément accessibles à l'homme de l'art par traitement alcalin d'un composé de formule (a) : 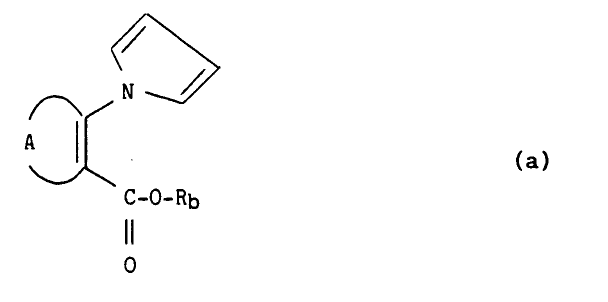 dans laquelle A est tel que défini dans la formule (I) et R_{b} représente un groupement alkyle (C₁-C₄),
ou par hydrolyse d'un composé de formule (b) : 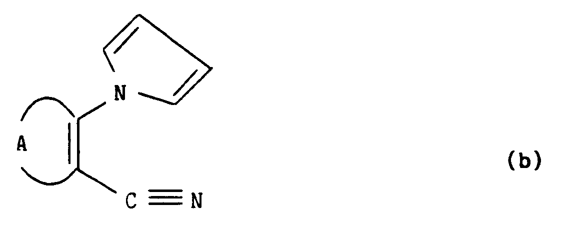 dans laquelle A est tel que défini précédemment,
pour conduire à un composé de formule (c) : 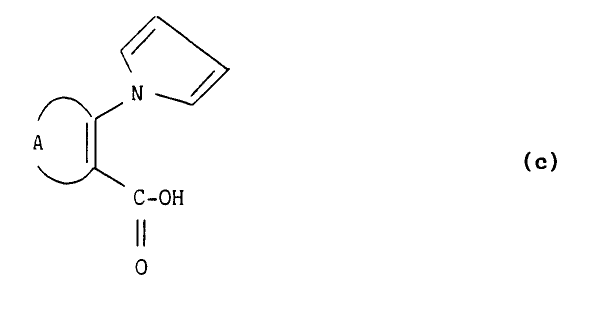 dans laquelle A est tel que défini précédemment,
composé de formule (c) qui est ensuite mis en réaction avec l'azoture de sodium pour obtenir le composé correspondant de formule (II) : 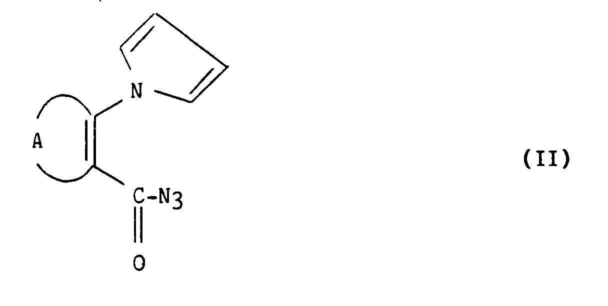 dans laquelle A est tel que défini précédemment,
les composés de formule (II) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques.

Les matières premières utilisées dans les procédés précédemment décrits sont :
- soit commerciales,
- soit aisément accessibles à l'homme de métier selon des procédés décrits dans la littérature.

Les composés de formule (I) possèdent des propriétés pharmacologiques très intéressantes.

Les composés de l'invention sont notamment de très puissants ligands sélectifs des récepteurs 5-HT₃ (voir les études de liaison aux récepteurs dans l'exemple A de la présente demande).

En outre, leur puissante affinité pour les récepteurs 5-HT₃ a été démontré aussi bien in vitro (mesure de l'accumulation de ¹⁴C-guanidinium dans les cellules NG 108-15, exemple B de la présente demande) qu'in vivo (mesure du réflexe de BEZOLD-JARISCH, exemple C de la présente demande) pour certains composés de la présente demande.

Les composés de l'invention sont donc susceptibles d'être utilisés dans la prévention et le traitement de l'anxiété, de la dépression, du stress, des psychoses, de la schizophrénie, des troubles du système nerveux central, de la migraine, des troubles de la mémoire, des troubles du comportement alimentaire, de l'alcoolisme, de la douleur, ainsi que dans la prévention et le traitement du vomissement et des désordres de la vidange gastrique.

L'invention s'étend aussi aux compositions pharmaceutiques renfermant comme principe actif au moins un des composés de formule (I) ou un de ses sels d'addition avec un acide ou avec une base pharmaceutiquement acceptable en combinaison avec un ou plusieurs excipients ou véhicules pharmaceutiquement acceptables.

Parmi les compositions selon l'invention, on pourra citer, à titre d'exemples et de façon non limitative, celles qui conviennent pour l'administration orale, parentérale, oculaire, per ou trans-cutanée, nasale, rectale, perlinguale, ou respiratoire, et notamment les préparations injectables, les aérosols, les gouttes oculaires ou nasales, les comprimés, les comprimés sublinguaux, les gélules, les capsules, les tablettes, les glossettes, les suppositoires, les crèmes, les pommades, et les gels.

Les préparations ainsi obtenues se présentent généralement sous forme dosée et peuvent contenir selon les affections traitées, l'âge, et le sexe du malade de 0,01 à 100 mg de principe actif en prises de une à trois fois par jour, de préférence de 0,01 à 5 mg de principe actif, particulièrement de 0,1 à 5 mg, par exemple 1 mg.

Les exemples qui suivent illustrent l'invention et ne la limitent en aucune façon.

### PREPARATION 1 : AZOTURE DE 3-(PYRROL-1-YL)-2-THENOYLE

### STADE A : 3-(pyrrol-1-yl)-2-thénoate de méthyle

On ajoute à froid par petites portions, en 5 minutes, 20 g (0,127 mole) de 3-amino-2-thénoate de méthyle à une solution de 16,80 g (0,127 mole) de 2,5-diméthoxytétrahydrofuranne dans 400 cm³ de dioxane en présence d'un équivalent de chlorhydrate de 4-chloropyridinium. La solution est portée au reflux 2 heures, puis concentrée sous pression réduite. Le résidu est repris dans 200 cm³ d'eau et extrait par 600 cm³ d'éther éthylique. La phase éthérée est séchée, décolorée au charbon animal, puis concentrée sous vide. L'huile résiduelle obtenue cristallise sous forme de cristaux blancs.
Solvant de recristallisation : éther éthylique-hexane (4/1)
Rendement : 68%
Point de fusion : 60°C
Caractéristiques spectrales :
   - Infrarouge :: ν CO : 1700 cm⁻¹
   ν C=C : 1545 cm⁻¹

### STADE B : acide 3-(pyrrol-1-yl)-2-thénoïque

Une solution de 20 g (0,096 mole) du composé obtenu au stade A dans un mélange de 150 cm³ de méthanol et de 100 cm³ d'une solution d'hydroxyde de sodium 6N est chauffée au reflux sous agitation pendant 3 heures, puis le méthanol est évaporé sous pression réduite. Le mélange réactionnel est dilué avec 100 cm³ d'eau acidifiée, puis extrait par 500 cm³ d'éther. La phase éthérée est décantée, séchée, puis concentrée sous vide. Le résidu est recristallisé dans l'éther éthylique pour obtenir l'acide 3-(pyrrol-1-yl)-2-thénoïque.
Point de fusion : 170°C
Rendement : 74%
Caractéristiques spectrales :
   - Infrarouge :: ν CO : 1675 cm⁻¹
   ν OH : 2980, 2520 cm⁻¹

### STADE C : azoture de 3-(pyrrol-1-yl)-2-thénoyle

On ajoute goutte à goutte, à 0°C, à une solution de 10 g (0,0518 mole) du composé obtenu au stade B, dans un mélange (50/50) d'acétone et 7,18 cm³ d'acétonitrile, 18 cm³ de triéthylamine. Après 30 minutes d'agitation, on ajoute, goutte à goutte, 4,97 cm3 de chloroformiate d'éthyle de façon à ce que la température reste comprise entre 0 et 5°C, puis après 30 minutes d'agitation, on ajoute 4,04 g (0,062 mole) d'azoture de sodium en solution dans 30 cm³ d'eau. Le mélange réactionnel est agité à 0°C pendant 2 heures puis versé sur 1 litre d'eau agitée, le précipité formé est essoré, lavé avec un minimum d'eau et séché à température ordinaire.
Solvant de recristallisation : éther éthylique
Rendement : 84%
Point de fusion : 100°C
Caractéristiques spectrales :
   - Infrarouge :: ν CON₃ : 2150 cm⁻¹
   ν CO : 1670 cm⁻¹
   ν principales : 1420, 1220, 1000, 780 et 740 cm⁻¹

### PREPARATIONS 2 A 7 :

En procédant comme dans la préparation 1, mais en remplaçant au stade A le 3-amino-2-thénoate de méthyle par le thénoate de méthyle convenablement substitué, on obtient les composés des préparations suivantes :

### PREPARATION 2 : AZOTURE DE 5-PHENYL-3-(PYRROL-1-YL)-2-THENOYLE

Solvant de recristallisation : éther
Point de fusion : 64°C

### PREPARATION 3 : AZOTURE DE 5-(4-FLUOROPHENYL)-3-(PYRROL-1-YL)-2-THENOYLE

Solvant de recristallisation : éther
Point de fusion : 108°C

### PREPARATION 4 : AZOTURE DE 4-PHENYL-3-(PYRROL-1-YL)-2-THENOYLE

Solvant de recristallisation : éther
Point de fusion : 122°C

### PREPARATION 5 : AZOTURE DE 4-(4-METHOXYPHENYL)-3-(PYRROL-1-YL)-2-THENOYLE

Solvant de recristallisation : éther
Point de fusion : 106°C

### PREPARATION 6 : AZOTURE DE 5-PHENYL-2-(PYRROL-1-YL)-3-THENOYLE

Solvant de recristallisation : éther
Point de fusion : 72°C

### PREPARATION 7 : AZOTURE DE 4,5-DIMETHYL-2-(PYRROL-1-YL)-3-THENOYLE

Point de fusion : huile

### PREPARATION 8 : AZOTURE DE 2-(PYRROL-1-YL)-3-THENOYLE

Sa préparation est décrite dans la publication de Rault et al., Hétérocycles, vol.14, n°5, 1980.

### EXEMPLE 1 :

### 5-(4-METHYLPIPERAZIN-1-YL)PYRROLO[1,2-a]THIENO[3,2-e]PYRAZINE

### STADE A : 5-oxo 4,5-dihydro-pyrrolo[1,2-a]thiéno[3,2-e]pyrazine

### Méthode A :

Une suspension de 10 g d'azoture de 2-(pyrrol-1-yl)-3-thénoyle dans 150 cm³ d'ortho-dichlorobenzene est chauffée au reflux pendant 1 heure. Après refroidissement le précipité est essoré, lavé avec de l'éther éthylique et séché pour obtenir la 5-oxo 4,5-dihydro-pyrrolo[1,2-a]thiéno[3,2-e] pyrazine.
Solvant de recristallisation : acétone (cristaux blancs)
Rendement : 92%
Point de fusion : 272°C

### Méthode B :

1 g d'azoture de 2-(pyrrol-1yl)-3-thénoyle est chauffé à sec à 130°C. Quand le dégagement gazeux a cessé, le résidu est sublimé à 180°C sous 0,04 mm Hg.
Rendement 82%

### STADE B : 5-chloro pyrrolo[1,2-a]thiéno[3,2-e]pyrazine

Une suspension de 6 g de 5-oxo 4,5-dihydro-pyrrolo[1,2-a]thiéno[3,2-e] pyrazine, obtenue au stade précédent, dans 300 cm³ d'oxychlorure de phosphore et 6 cm³ de pyridine, est chauffée au reflux pendant 4 heures. L'oxychlorure de phosphore est ensuite éliminé sous vide, le résidu est trituré avec une solution d'ammoniaque à 10%, essoré, lavé à l'eau et séché pour obtenir la 5-chloropyrrolo[1,2-a]thiéno[3,2-e]pyrazine.
Solvant de recristallisation : acétone (cristaux jaunes pâles sublimables à 120°C sous 0,04 mm Hg)
Rendement : 62%
Point de fusion : 127°C

### STADE C : 5-(4-méthylpipérazin-1-yl)pyrrolo[1,2-a]thiéno[3,2-e]pyrazine

A une solution de 0,010 mole (2,08 g) de 5-chloropyrrolo[1,2-a]thiéno[3,2-e]pyrazine dans 10 cm³ de diméthylformamide, on additionne 0,011 mole de 1-méthylpipérazine et 0,020 mole (2,12 g) de carbonate de sodium. Le mélange réactionnel est chauffé à reflux pendant 2 heures puis refroidi et additionné de 100 cm³ d'eau. La suspension ainsi obtenue est extraite 3 fois avec 100 cm³ d'éther éthylique. Les phases organiques sont réunies et lavées 3 fois avec 100 cm³ d'eau puis séchées sur sulfate de magnésium et décolorées sur charbon animal. Le solvant est éliminé sous pression réduite et l'huile résiduelle obtenue cristallise par refroidissement pour donner la 5-(4-méthylpipérazin-1-yl)pyrrolo[1,2-a]thiéno[3,2-e]pyrazine. 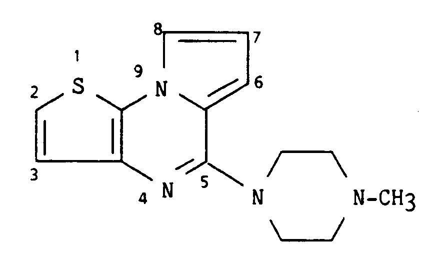
Solvant de recristallisation : isopropanol
Rendement : 79%
Point de fusion : 82°C
Caractéristiques spectrales :
   - Infrarouge :: ν C-H : 3100, 2940, et 2800 cm⁻¹
   ν principales : 1570, 1500, 1270, 1180, 1140, 1010, 960, 870, 785, 740, 710, et 640 cm⁻¹
RMN ¹H (δ ppm) tétraméthylsilane (TMS) (DMSO-d₆) : 7,73 (H₈) ; 7,34 (H₂) ; 7,22 (H₃) ; 6,89 (H₆) ; 6,82 (H₇) ; 3,57 et 2,50 (CH₂) ; 2,24 (CH₃)

### EXEMPLE 2 :

### FUMARATE DE 5-(4-METHYLPIPERAZIN-1-YL)PYRROLO[1,2-a]THIENO[3,2-e]PYRAZINE

On additionne à chaud à une solution de 0,010 mole du composé obtenu à l'exemple 1 dans 15 cm³ d'acétone anhydre, 0,011 mole (1,2 g) d'une solution d'acide fumarique dans 75 cm³ d'acétone anhydre. Le mélange réactionnel est chauffé à reflux pendant 15 minutes puis on laisse refroidir jusqu'à température ambiante. Le fumarate attendu cristallise dans ce milieu, il est récupéré par filtration puis séché et recristallisé dans l'isopropanol.
Rendement : 91%
Point de fusion : 200°C
Caractéristiques spectrales :
   - Infrarouge :: ν N⁺- : 2700 cm⁻¹ (large)
   ν C-H : 3100, 2920 et 2840 cm⁻¹
   ν C=O : 1680 cm⁻¹
   ν principales : 1480, 1410, 1385, 1280, 1180, 1140, 1100, 1050, 1025, 985, 950, 740 et 650 cm⁻¹

### EXEMPLE 3 :

### 5-(4-METHYLPIPERAZIN-1-YL)PYRROLO[1,2-a]THIENO[2,3-e]PYRAZINE

En procédant comme dans l'exemple 1, mais en remplaçant au stade A l'azoture de 2-(pyrrol-1-yl)-3-thénoyle par l'azoture de 3-(pyrrol-1-yl)-2-thénoyle, on obtient successivement les composés des stades suivants :

### STADE A : 5-oxo 4,5-dihydro-pyrrolo[1,2-a]thiéno[2,3-e]pyrazine

Rendement : 81%
Point de fusion : 276°C

### STADE B : 5-chloropyrrolo[1,2-a]thiéno[2,3-e]pyrazine

Solvant de recristallisation : éther éthylique
Rendement : 61%
Point de fusion : 180°C

### STADE C : 5-(4-méthylpipérazin-1-yl)pyrrolo[1,2-a]thiéno[2,3-e]pyrazine

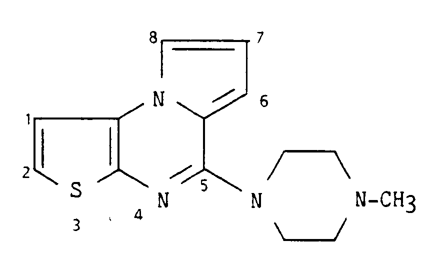

Solvant de recristallisation : isopropanol
Rendement : 81%
Point de fusion : 98°C
Caractéristiques spectrales :
   - Infrarouge :: ν C-H : 3100, 2960 et 2800 cm⁻¹
   ν principales : 1500, 1450, 1410, 1360, 1260, 1180, 1140, 1015, 950, 800 et 710 cm⁻¹
RMN ¹H (δ ppm) tétraméthylsilane (TMS) (DMSO-d₆) : 7,54 (H₈) ; 7,27 (H₂) ; 7,11 (H₁) ; 6,76 (H₇ et H₆) ; 3,75 et 2,63 (CH₂) ; 2,38 (CH₃)

### EXEMPLE 4 :

### FUMARATE DE 5-(4-METHYLPIPERAZIN-1-YL)PYRROLO[1,2-a]THIENO[2,3-e]PYRAZINE

En procédant comme dans l'exemple 2, mais en remplaçant le composé de l'exemple 1 par le composé de l'exemple 3, on obtient le fumarate de 5-(4-méthylpipérazin-1-yl)pyrrolo[1,2-a]thiéno[2,3-e]pyrazine.
Rendement : 93%
Point de fusion : 190°C
Caractéristiques spectrales :
   - Infrarouge :: ν C-H : 3100 cm⁻¹
   ν =N- : 2700, 2300 cm⁻¹
   ν C=O : 1680 cm⁻¹
   ν principales : 1600, 1450, 1350, 1270, 1180, 1150, 1070, 980, 930, 860, 760, 720, 680 et 640 cm⁻¹

### EXEMPLES 5 ET 6 :

En procédant comme dans les exemples 1 et 2, mais en remplaçant au stade C de l'exemple 1 la 1-méthylpipérazine par la pipérazine, on obtient successivement les composés des exemples suivants :

### EXEMPLE 5 :

### 5-(PIPERAZIN-1-YL)PYRROLO[1,2-a]THIENO[3,2-e]PYRAZINE

Rendement : 92%
Point de fusion : 100°C
Caractéristiques spectrales :
   - Infrarouge :: ν N-H : 3200 cm⁻¹
   ν C-H : 2920 et 2820 cm⁻¹
   ν principales : 1570, 1520, 1485, 1420, 1270, 1130, 950, 890,870, 810, 740 et 700 cm⁻¹

### EXEMPLE 6 :

### FUMARATE DE 5-(PIPERAZIN-1-YL)PYRROLO[1,2-a]THIENO[3,2-e]PYRAZINE

Rendement : 96%
Point de fusion : 220°C
Caractéristiques spectrales :
   - Infrarouge :: ν NH⁺-, C-H : 3100-2400 cm⁻¹
   ν C=O : 1700 cm⁻¹
   ν principales : 1570, 1490, 1420, 1380, 1280, 1185, 1135, 1050, 1000, 955, 795, 740, 700 et 640 cm⁻¹

### EXEMPLES 7 ET 8 :

En procédant comme dans les exemples 1 et 2, mais en remplaçant au stade C de l'exemple 1 la 1-méthylpipérazine par la 1-benzylpipérazine, on obtient successivement les composés des exemples suivants :

### EXEMPLE 7 :

### 5-(4-BENZYLPIPERAZIN-1-YL)PYRROLO[1,2-a]THIENO[3,2-e]PYRAZINE

Rendement : 77%
Point de fusion : 84°C
Caractéristiques spectrales :
   - Infrarouge :: ν C-H : 3100, 3030, 2940 et 2820 cm⁻¹
   ν principales : 1480, 1460, 1410, 1370, 1260, 1150, 1010, 950, 820, 800, 740 et 700 cm⁻¹

### EXEMPLE 8 :

### FUMARATE DE 5-(4-BENZYLPIPERAZIN-1-YL)PYRROLO[1,2-a]THIENO[3,2-e]PYRAZINE

Rendement : 95%
Point de fusion : 192°C
Caractéristiques spectrales :
   - Infrarouge :: ν C-H : 3120, 3000, 2920 et 2880 cm⁻¹
   ν =N⁺- : 2680-2400 cm⁻¹
   ν C=O : 1700 cm⁻¹
   ν principales : 1640, 1570, 1490, 1390, 1270, 1200,1110, 990, 950, 750, 710 et 650 cm⁻¹

### EXEMPLES 9 ET 10 :

En procédant comme dans les exemples 1 et 2, mais en remplaçant au stade C la 1-méthylpipérazine par la 1-[(pyrrolidin-1-yl)carbonylméthyl] pipérazine, on obtient successivement les composés des exemples suivants :

### EXEMPLE 9 :

### 5-[4-(PYRROLIDIN-1-YLCARBONYLMETHYL)PIPERAZIN-1-YL]PYRROLO[1,2-a]THIENO [3,2-e]PYRAZINE

Rendement : 69%
Point de fusion : 70°C

### EXEMPLE 10 :

### FUMARATE DE 5-[4-(PYRROLIDIN-1-YLCARBONYLMETHYL)PIPERAZIN-1-YL]PYRROLO [1,2-a]THIENO[3,2-e]PYRAZINE

Rendement : 91%
Point de fusion : 194°C

### EXEMPLES 11 ET 12 :

En procédant comme dans les exemples 1 et 2, mais en remplaçant au stade C de l'exemple 1 la 1-méthylpipérazine par la 1-(4-chlorobenzhydryl) pipérazine, on obtient successivement les composés des exemples suivants :

### EXEMPLE 11 :

### 5-{4-[1-(4-CHLOROPHENYL)1-(PHENYL)METHYL]PIPERAZIN-1-YL}PYRROLO[1,2-a] THIENO[3,2-e]PYRAZINE

Rendement : 65%
Point de fusion : 140°C
Caractéristiques spectrales :
   - Infrarouge :: ν C-H : 3100, 2980, 2840 et 2800 cm⁻¹
   ν principales : 1480, 1410, 1370, 1250, 1160, 1140, 1090, 1000, 950, 870, 760, 740, 720 et 700 cm⁻¹

### EXEMPLE 12 :

### FUMARATE DE 5-{4-[1-(4-CHLOROPHENYL)1-(PHENYL)METHYL]PIPERAZIN-1-YL} PYRROLO[1,2-a]THIENO[3,2-e]PYRAZINE

Rendement : 91%
Point de fusion : 176°C

### EXEMPLE 13 :

### 5-(1,2,5,6-TETRAHYDROPYRIDIN-1-YL)PYRROLO[1,2-a]THIENO[3,2-e]PYRAZINE

En procédant comme dans l'exemple 1, mais en remplaçant au stade C de l'exemple 1 la 1-méthylpipérazine par la 1,2,5,6-tétrahydropyridine, on obtient la 5-(1,2,5,6-tétrahydropyridin-1-yl)pyrrolo[1,2-a]thiéno[3,2-e] pyrazine.
Rendement : 90%
Point de fusion : 82°C
Caractéristiques spectrales :
   - Infrarouge :: ν C-H : 3000, 2900 et 2800 cm⁻¹
   ν principales : 1470, 1410, 1370, 1350, 1250, 1135, 1080, 1030, 1010, 940, 790, 700, 640 et 620 cm⁻¹

### EXEMPLE 14 :

### CHLORHYDRATE DE 5-(1,2,5,6-TETRAHYDROPYRIDIN-1-YL)PYRROLO[1,2-a]THIENO [3,2-e]PYRAZINE

Le composé de l'exemple 13 est mis en solution dans une quantité minimale d'isopropanol à chaud puis on ajoute quelques gouttes d'acide chlorhydrique concentré. Le précipité observé au bout de quelques minutes est essoré, séché et recristallisé dans l'isopropanol pour obtenir le chlorhydrate de 5-(1,2,5,6-tétrahydropyridin-1-yl)pyrrolo[1,2-a]thiéno[3,2-e] pyrazine.
Rendement : 94%
Point de fusion : 218°C
Caractéristiques spectrales :
   - Infrarouge :: ν C-H : 3020 et 2880 cm⁻¹
   ν =N⁺- : 2800-2600 cm⁻¹
   ν principales : 1580, 1500, 1425, 1380, 1350, 1260, 1090, 1030, 955, 930, 880, 830, 700 et 620 cm⁻¹

### EXEMPLES 15 A 37 :

En procédant comme dans l'exemple 1, mais en remplaçant au stade C de l'exemple 1 la 1-méthylpipérazine par les amines appropriées puis, le cas échéant, en réalisant à la suite le sel des composés obtenus avec un acide pharmaceutiquement acceptable, on obtient les composés des exemples suivants :

### EXEMPLE 15 :

### 5-(1,4-THIOMORPHOLIN-4-YL)PYRROLO[1,2-a]THIENO[3,2-e]PYRAZINE

Rendement : 89%
Point de fusion : 116°C

### EXEMPLE 16 :

### CHLORHYDRATE DE 5-(1,4-THIOMORPHOLIN-4-YL)PYRROLO[1,2-a]THIENO[3,2-e] PYRAZINE

Rendement : 93%
Point de fusion : 238°C

### EXEMPLE 17 :

### 5-(4-PHENYLPIPERIDIN-1-YL)PYRROLO[1,2-a]THIENO[3,2-e]PYRAZINE

Rendement : 86%
Point de fusion : 108°C

### EXEMPLE 18 :

### CHLORHYDRATE DE 5-(4-PHENYLPIPERIDIN-1-YL)PYRROLO[1,2-a]THIENO[3,2-e] PYRAZINE

Rendement : 95%
Point de fusion : 250°C

### EXEMPLE 19 :

### 5-(4-PHENYLPIPERAZIN-1-YL)PYRROLO[1,2-a]THIENO[3,2-e]PYRAZINE

Rendement : 83%
Point de fusion : 140°C

### EXEMPLE 20 :

### DICHLORHYDRATE DE 5-(4-PHENYLPIPERAZIN-1-YL)PYRROLO[1,2-a]THIENO[3,2-e] PYRAZINE

Rendement : 96%
Point de fusion : 248°C

### EXEMPLE 21 :

### 5-[4-(3-TRIFLUOROMETHYLPHENYL)PIPERAZIN-1-YL]PYRROLO[1,2-a]THIENO[3,2-e] PYRAZINE

Rendement : 87%
Point de fusion : 128°C

### EXEMPLE 22 :

### CHLORHYDRATE DE 5-[4-(3-TRIFLUOROMETHYLPHENYL)PIPERAZIN-1-YL]PYRROLO[1,2-a]THIENO[3,2-e]PYRAZINE

Rendement : 92%
Point de fusion : 208°C

### EXEMPLE 23 :

### 5-[4-(ETHOXYCARBONYL)PIPERAZIN-1-YL]PYRROLO[1,2-a]THIENO[3,2-e]PYRAZINE

Rendement : 85%
Point de fusion : 104°C

### EXEMPLE 24 :

### CHLORHYDRATE DE 5-[4-(ETHOXYCARBONYL)PIPERAZIN-1-YL]PYRROLO[1,2-a]THIENO [3,2-e]PYRAZINE

Rendement : 93%
Point de fusion : 194°C

### EXEMPLE 25 :

### 5-[4-(PYRID-2-YL)PIPERAZIN-1-YL]PYRROLO[1,2-a]THIENO[3,2-e]PYRAZINE

Rendement : 68%
Point de fusion : 118°C
Caractéristiques spectrales :
   - Infrarouge :: ν C-H : 2800 cm⁻¹
   ν principales : 1590, 1460, 1480, 1430, 1310, 1250, 1140, 1040, 1000, 950, 770, 730, 700 et 640 cm⁻¹

### EXEMPLE 26 :

### 5-[4-(2,4-DICHLOROBENZYL)PIPERAZIN-1-YL]PYRROLO[1,2-a]THIENO[3,2-e] PYRAZINE

Rendement : 82%
Point de fusion : 76°C

### EXEMPLE 27 :

### DICHLORHYDRATE DE 5-[4-(2,4-DICHLOROBENZYL)PIPERAZIN-1-YL]PYRROLO[1,2-a] THIENO[2,3-e]PYRAZINE

Rendement : 92%
Point de fusion : 198°C

### EXEMPLE 28 :

### 5-[4-(3,4-DICHLOROBENZYL)PIPERAZIN-1-YL]PYRROLO[1,2-a]THIENO[3,2-e] PYRAZINE

Rendement : 76%
Point de fusion : Huile

### EXEMPLE 29 :

### FUMARATE DE 5-[4-(3,4-DICHLOROBENZYL)PIPERAZIN-1-YL]PYRROLO[3,2-a]THIENO [2,3-e]PYRAZINE

Rendement : 84%
Point de fusion : 188°C

### EXEMPLE 30 :

### 5-[4-(4-CHLOROPHENYL)PIPERAZIN-1-YL]PYRROLO[1,2-a]THIENO [3,2-e]PYRAZINE

Rendement : 82%
Point de fusion : 128°C

### EXEMPLE 31 :

### DICHLORHYDRATE DE 5-[4-(4-CHLOROPHENYL)PIPERAZIN-1-YL]PYRROLO[1,2-a]THIENO [3,2-e]PYRAZINE

Rendement : 92%
Point de fusion : 170°C

### EXEMPLE 32 :

### 5-[4-(4-FLUOROPHENYL)PIPERAZIN-1-YL]PYRROLO[1,2-a]THIENO[3,2-e]PYRAZINE

Rendement : 68%
Point de fusion : 114°C

### EXEMPLE 33 :

### DICHLORHYDRATE DE 5-[4-(4-FLUOROPHENYL)PIPERAZIN-1-YL]PYRROLO[1,2-a] THIENO[3,2-e]PYRAZINE

Rendement : 93%
Point de fusion : 174°C

### EXEMPLE 34 :

### 5-(4-PROPYLPIPERAZIN-1-YL)PYRROLO[1,2-a]THIENO[3,2-e]PYRAZINE

Rendement : 67%
Point de fusion : Huile

### EXEMPLE 35 :

### DICHLORHYDRATE DE 5-(4-PROPYLPIPERAZIN-1-YL)PYRROLO[1,2-a]THIENO[3,2-e] PYRAZINE

Rendement : 88%
Point de fusion : > 260°C

### EXEMPLE 36 :

### 5-(4-ALLYLPIPERAZIN-1-YL)PYRROLO[1,2-a]THIENO[3,2-e]PYRAZINE

Rendement : 78%
Point de fusion : Huile

### EXEMPLE 37 :

### DICHLORHYDRATE DE 5-(4-ALLYLPIPERAZIN-1-YL)PYRROLO[1,2-a]THIENO[3,2-e] PYRAZINE

Rendement : 94%
Point de fusion : 254°C

### EXEMPLES 38 A 41 :

En procédant comme dans l'exemple 1, mais en remplaçant au stade A l'azoture de 2-(pyrrol-1-yl)-3-thénoyle par l'azoture de 3-(pyrrol-1-yl)-2-thénoyle et en remplaçant au stade C la 1-méthylpipérazine par les amines appropriées, on obtient les composés des exemples suivants :

### EXEMPLE 38 :

### 5-(4-BENZYLPIPERAZIN-1-YL)PYRROLO[1,2-a]THIENO[2,3-e]PYRAZINE

Point de fusion (trichlorhydrate) : 163°C

### EXEMPLE 39 :

### 5-[4-(3-TRIFLUOROMETHYLPHENYL)PIPERAZIN-1-YL]PYRROLO[1,2-a]THIENO[2,3-e] PYRAZINE

Point de fusion (monochlorhydrate) : 195°C

### EXEMPLE 40 :

### 5-[4-(3,4-DICHLOROBENZYL)PIPERAZIN-1-YL]PYRROLO[1,2-a]THIENO[2,3-e] PYRAZINE

Point de fusion (trichlorhydrate) : 177°C
Caractéristiques spectrales :
   - Infrarouge :: ν principales : 3400, 3080, 1590, 1420, 1310, 940 et 700 cm⁻¹

### EXEMPLE 41 :

### 5-{4[4,4-bis(4-CHLORO-PHENYL)BUTYL]PIPERAZIN-1-YL}PYRROLO[1,2-a]THIENO[2,3-e]PYRAZINE

Point de fusion (trichlorhydrate) : 140°C
Caractéristiques spectrales :
   - Infrarouge :: ν principales : 1590, 1495, 1210, 1150 et 825 cm⁻¹

### EXEMPLE 42 :

### 5-(4-ALLYLPIPERAZIN-1-YL)PYRROLO[1,2-a]THIENO[2,3-e]PYRAZINE

Point de fusion (dichlorhydrate) : 220°C

### EXEMPLE 43 :

### 5-[4-(2,4-DICHLOROBENZYL)PIPERAZIN-1-YL]PYRROLO[1,2-a]THIENO[2,3-e] PYRAZINE

Point de fusion (dichlorhydrate) : 155°C

### EXEMPLE 44 : (second procédé)

### 5-[4-(3-TRIFLUOROMETHYLPHENYL)PIPERAZIN-1-YL]PYRROLO[1,2-a]THIENO[2,3-e] PYRAZINE

### STADE A : N-[3-(pyrrol-1-yl)thién-2-yl] [4-(3-trifluorométhylphényl) pipérazin-1-yl]carboxamide

On porte au reflux 2 heures dans 70 cm³ de benzène 3 g (0,0137 mole) d'azoture de 3-(pyrrol-1-yl)-2-thénoyle (obtenu dans la préparation 1) et 3,15 g (0,0137 mole) de 1-(3-trifluorométhylphényl)pipérazine. Après refroidissement, la solution benzénique est concentrée sous pression réduite. L'huile résiduelle cristallise par addition d'éther éthylique (poudre rose).
Rendement : 90%
Point de fusion : 98°C
Caractéristiques spectrales :
   - Infrarouge :: ν NH : 3260 cm⁻¹
   ν CO : 1640 cm⁻¹

### STADE B : 5-[4-(3-trifluorométhylphényl)pipérazin-1-yl]pyrrolo[1,2-a] thiéno[2,3-e]pyrazine

L'huile résiduelle obtenue au stade précédent est agitée dans 30 cm³ d'oxychlorure de phosphore puis portée au reflux pendant 2 heures. La solution est concentrée sous vide et le résidu est dilué dans 150 cm³ d'une solution d'hydrogénocarbonate de sodium puis extrait par 150 cm³ d'acétate d'éthyle. La phase organique est lavée à l'eau, décantée, séchée puis concentrée sous pression réduite pour obtenir le 5-[4-(3-trifluorométhylphényl)pipérazin-1-yl]pyrrolo[1,2-a]thiéno[2,3-e]pyrazine.

Le sel de la 5-[4-(3-trifluorométhylphényl)pipérazin-1-yl]pyrrolo[1,2-a] thiéno[2,3-e]pyrazine avec l'acide chlorhydrique est obtenu en dissolvant le composé du titre dans 30 cm³ d'isopropanol puis en acidifiant par 1 cm³ d'acide chlorhydrique concentré 10N. Après 10 minutes d'agitation, le précipité est essoré, lavé à l'éther éthylique, seché et recristallisé dans l'acétonitrile.
Point de fusion (dichlorhydrate) : 195°C
Caractéristiques spectrales :
   - Infrarouge :: ν =NH⁺- : 2720, 2420 cm⁻¹
   ν C=C, C=N : 1595 cm⁻¹

### EXEMPLES 45 A 69 :

En procédant comme dans l'exemple 44, mais en remplaçant au stade A, l'azoture de 3-(pyrrol-1-yl)-2-thénoyle par l'azoture de thénoyle convenablement substitué et en utilisant, au stade A, l'amine appropriée, on obtient les composés des exemples suivants :

### EXEMPLE 45 : (second procédé)

### 5-(4-BENZYLPIPERAZIN-1-YL)PYRROLO[1,2-a]THIENO[2,3-e]PYRAZINE

### STADE A : N-[3-(pyrrol-1-yl)thién-2-yl] (4-benzylpipérazin-1-yl) carboxamide

Point de fusion : 158°C

### STADE B : 5-(4-benzylpipérazin-1-yl)pyrrolo[1,2-a]thiéno[2,3-e]pyrazine

Point de fusion (trichlorhydrate) : 163°C

### EXEMPLE 46 :

### 2-PHENYL 5-[4-(3-TRIFLUOROMETHYLPHENYL)PIPERAZIN-1-YL]PYRROLO[1,2-a] THIENO[2,3-e]PYRAZINE

### STADE A : N-[5-phényl 3-(pyrrol-1-yl)thién-2-yl] [4-(3-trifluorométhyl phényl)pipérazin-1-yl]carboxamide

Point de fusion : 129°C

### STADE B : 2-phényl 5-[4-(3-trifluorométhylphényl)piperazin-1-yl]pyrrolo [1,2-a]thiéno[2,3-e]pyrazine

Point de fusion (monochlorhydrate) : 160°C

### EXEMPLE 47:

### 2-PHENYL 5-(4-BENZYLPIPERAZIN-1-YL)PYRROLO[1,2-a]THIENO[2,3-e]PYRAZINE

### STADE A : N-[5-phényl 3-(pyrrol-1-yl)thién-2-yl] [4-benzylpipérazin-1-yl]

Point de fusion : 160°C

### STADE B : 2-phényl 5-(4-benzylpipérazin-1-yl)pyrrolo[1,2-a]thiéno[2,3-e] pyrazine

Point de fusion (trichlorhydrate) : 182°C

### EXEMPLE 48 :

### 2-(4-FLUOROPHENYL) 5-[4-(3-TRIFLUOROMETHYLPHENYL)PIPERAZIN-1-YL]PYRROLO [1,2-a]THIENO[2,3-e]PYRAZINE

### STADE A : N-[5-(4-fluorophényl) 3-(pyrrol-1-yl)thién-2-yl] [4-(3-trifluorométhylphényl)pipérazin-1-yl]carboxamide

Point de fusion : 134°C

### STADE B : 2-(4-fluorophényl) 5-[4-(3-trifluorométhylphényl)piperazin-1-yl]pyrrolo[1,2-a]thiéno[2,3-e]pyrazine

Point de fusion (dichlorhydrate) : 212°C

### EXEMPLE 49 :

### 1-PHENYL 5-[4-(3-TRIFLUOROMETHYLPHENYL)PIPERAZIN-1-YL]PYRROLO[1,2-a] THIENO[2,3-e]PYRAZINE

### STADE A : N-[4-phényl 3-(pyrrol-1-yl)thién-2-yl] [4-(3-trifluorométhyl phényl)pipérazin-1-yl]carboxamide

Point de fusion : 192°C

### STADE B : 1-phényl 5-[4-(3-trifluorométhylphényl)pipérazin-1-yl]pyrrolo [1,2-a]thiéno[2,3-e]pyrazine

Point de fusion (monochlorhydrate) : 130°C

### EXEMPLE 50 :

### 1-PHENYL 5-[4-BENZYLPIPERAZIN-1-YL]PYRROLO[1,2-a]THIENO[2,3-e]PYRAZINE

### STADE A : N-[4-phényl 3-(pyrrol-1-yl)thién-2-yl] [4-benzylpipérazin-1-yl]carboxamide

Point de fusion : 175°C

### STADE B : 1-phényl 5-[4-benzylpipérazin-1-yl]pyrrolo[1,2-a]thiéno[2,3-e] pyrazine

Point de fusion (trichlorhydrate) : 210°C

### EXEMPLE 51 :

### 1-(4-METHOXYPHENYL) 5-[4-(3-TRIFLUOROMETHYLPHENYL)PIPERAZIN-1-YL]PYRROLO [1,2-a]THIENO[2,3-e]PYRAZINE

### STADE A : N-[4-(4-méthoxyphényl) 3-(pyrrol-1-yl)thién-2-yl] [4-(3-trifluorométhylphényl)pipérazin-1-yl]carboxamide

Point de fusion : 166°C

### STADE B : 1-(4-méthoxyphényl) 5-[4-(3-trifluorométhylphényl)pipérazin-1-yl]pyrrolo[1,2-a]thiéno[2,3-e]pyrazine

Point de fusion (monochlorhydrate) : 162°C

### EXEMPLE 52 :

### 2-PHENYL 5-[4-(3-TRIFLUOROMETHYLPHENYL)PIPERAZIN-1-YL]PYRROLO[1,2-a] THIENO[3,2-e]PYRAZINE

### STADE A : N-[5-phényl 2-(pyrrol-1-yl)thién-3-yl] [4-(3-trifluorométhyl phényl)pipérazin-1-yl]carboxamide

Point de fusion : 160°C

### STADE B : 2-phényl 5-[4-(3-trifluorométhylphényl)pipérazin-1-yl]pyrrolo[1,2-a] thiéno[3,2-e]pyrazine

Point de fusion (dichlorhydrate) : 135°C

### EXEMPLE 53 :

### 2-PHENYL 5-(4-METHYLPIPERAZIN-1-YL)PYRROLO[1,2-a]THIENO[3,2-e]PYRAZINE

### STADE A : N-[5-phényl 2-(pyrrol-1-yl)thién-3-yl] [4-méthylpipérazin-1-yl]carboxamide

Huile brune

### STADE B : 2-phényl 5-(4-méthylpipérazin-1-yl)pyrrolo[1,2-a]thiéno[3,2-e] pyrazine

Point de fusion (dichlorhydrate) : > 260°C

### EXEMPLE 54:

### 2-PHENYL 5-(4-BENZYLPIPERAZIN-1-YL)PYRROLO[1,2-a]THIENO[3,2-e]PYRAZINE

### STADE A : N-[5-phényl 2-(pyrrol-1-yl)thién-3-yl] [4-benzylpipérazin-1-yl]carboxamide

Huile brune

### STADE B : 2-phényl 5-(4-benzylpipérazin-1-yl)pyrrolo[1,2-a]thiéno[3,2-e] pyrazine

Point de fusion (dichlorhydrate) : 186°C

### EXEMPLE 55 :

### 2-PHENYL 5-[4-(ETHOXYCARBONYL)PIPERAZIN-1-YL]PYRROLO[1,2-a]THIENO[3,2-e] PYRAZINE

### STADE A : N-[5-phényl 2-(pyrrol-1-yl)thién-3-yl] [4-(éthoxycarbonyl) pipérazin-1-yl]carboxamide

Huile brune

### STADE B : 2-phényl 5-[4-(éthoxycarbonyl)pipérazin-1-yl]pyrrolo[1,2-a] thiéno[3,2-e]pyrazine

Point de fusion : 136°C

### EXEMPLE 56 :

### 2-PHENYL 5-(PIPERAZIN-1-YL)PYRROLO[1,2-a]THIENO[3,2-e]PYRAZINE

### STADE A : 2-phényl 5-(pipérazin-1-yl)pyrrolo[1,2-a]thiéno[3,2-e] pyrazine

Point de fusion (trichlorhydrate) : 206°C

### EXEMPLE 57 :

### 2,3-DIMETHYL 5-(4-BENZYLPIPERAZIN-1-YL)PYRROLO[1,2-a]THIENO[3,2-e] PYRAZINE

### STADE A : N-[4,5-diméthyl 2-(pyrrol-1-yl)thién-3-yl] (4-benzylpipérazin-1-yl)carboxamide

Point de fusion : 168°C

### STADE B : 2,3-diméthyl 5-(4-benzylpipérazin-1-yl)pyrrolo[1,2-a]thiéno [3,2-e]pyrazine

Point de fusion (trichlorhydrate) : 164°C

### EXEMPLE 58 :

### 2-PHENYL 5-(4-METHYLPIPERAZIN-1-YL)PYRROLO[1,2-a]THIENO[2,3-e]PYRAZINE

Point de fusion (trichlorhydrate) : 196°C

### EXEMPLE 59 :

### 5-(4-PHENYLPIPERIDIN-1-YL)PYRROLO[1,2-a]THIENO[2,3-e]PYRAZINE

Point de fusion (chlorhydrate) : 185°C

### EXEMPLE 60 :

### 5-[4-(3,4-DICHLOROPHENYL)PIPERAZIN-1-YL]PYRROLO[1,2-a]THIENO[3,2-e] PYRAZINE

### EXEMPLE 61 :

### 5-[4-(4-METHOXYPHENYL)PIPERAZIN-1-YL]PYRROLO[1,2-a]THIENO [3,2-e]PYRAZINE

### EXEMPLE 62 :

### 5-(2,6-DIMETHYLMORPHOLIN-4-YL)PYRROLO[1,2-a]THIENO[3,2-e]PYRAZINE

### EXEMPLE 63 :

### 5-{[2-(BENZYLAMINO)ETHYL]AMINE}PYRROLO[1,2-a]THIENO[3,2-e]PYRAZINE

### EXEMPLE 64 :

### 2-PHENYL 5-[2,5-DIMETHYLPIPERAZIN-1-YL]PYRROLO[1,2-a]THIENO[3,2-e] PYRAZINE

### EXEMPLE 65 :

### 2-PHENYL 5-[4-(4-NITROPHENYL)PIPERAZIN-1-YL]PYRROLO[1,2-a]THIENO[3,2-e] PYRAZINE

### EXEMPLE 66 :

### 5-(3,3,5-TRIMETHYLPERHYDROAZEPIN-1-YL)PYRROLO[1,2-a]THIENO[3,2-e]PYRAZINE

### EXEMPLE 67 :

### 5-(HOMOPIPERAZIN-1-YL)PYRROLO[1,2-a]THIENO[3,2-e]PYRAZINE

### EXEMPLE 68 :

### 5-[4-(4-CHLOROPHENYL) 4-HYDROXYPIPERIDIN-1-YL]PYRROLO[1,2-a]THIENO[3,2-e] PYRAZINE

### EXEMPLE 69 :

### 2-PHENYL 5-[4-(4-FLUOROPHENYL) 4-HYDROXYPIPERIDIN-1-YL]PYRROLO[1,2-a] THIENO[3,2-e]PYRAZINE

### EXEMPLE 70 :

### 5-OXO 1-PHENYL 4,5-DIHYDRO-PYRROLO[1,2-a]THIENO[2,3-e]PYRAZINE

On ajoute par petites fractions 1 g (0,0034 mole) d'azoture de 4-phényl-3-(pyrrol-1-yl)-2-thénoyle dans 30 cm³ d'ortho-dichlorobenzène chauffé à 180°C. Après 5 minutes à cette température, la solution est refroidie. Le précipité formé est essoré, lavé à l'éther éthylique et séché.
Rendement : 77%
Point de fusion : 250°C
Caractéristiques spectrales :
   - Infrarouge :: ν NH : 3120, 2900 cm⁻¹
   ν CO : 1625 cm⁻¹

### EXEMPLE 71 :

### 5-OXO 2-PHENYL 4,5-DIHYDRO-PYRROLO[1,2-a]THIENO[2,3-e]PYRAZINE

En procédant comme dans l'exemple 70, mais en remplaçant l'azoture de 4-phényl 3-(pyrrol-1-yl)-2-thénoyle par l'azoture de 5-phényl 3-(pyrrol-1-yl)-2-thénoyle, on obtient la 5-oxo 2-phényl 4,5-dihydro-pyrrolo[1,2-a] thiéno[2,3-e]pyrazine
Point de fusion : 291°C
Caractéristiques spectrales :
   - Infrarouge :: ν (NH) : 3120, 2800 cm⁻¹
   ν CO : 1650 cm⁻¹

### EXEMPLE 72 :

### 5-OXO 2-PHENYL 4,5-DIHYDRO-PYRROLO[1,2-a]THIENO[3,2-e]PYRAZINE

En procédant comme dans l'exemple 70, mais en remplaçant l'azoture de 4-phényl 3-(pyrrol-1-yl)-2-thénoyle par l'azoture de 5-phényl 2-(pyrrol-1-yl)-3-thénoyle, on obtient la 5-oxo 2-phényl 4,5-dihydro-pyrrolo[1,2-a] thiéno[3,2-e]pyrazine
Rendement : 78%
Point de fusion : 255°C
Caractéristiques spectrales :
   - Infrarouge :: ν NH : 3100, 2800 cm⁻¹
   ν CO : 1640 cm⁻¹

### EXEMPLES 73 A 78 :

En procédant comme dans les exemples 1 ou 3, mais en remplaçant au stade C, la 1-méthylpipérazine par les amines appropriées, on obtient les composés des exemples suivants :

### EXEMPLE 73 :

### 5-[4-(4-FLUOROBENZYL)PIPERAZIN-1-YL]-4H-PYRROLO[1,2-a]THIENO[2,3-e] PYRAZINE

Point de fusion (dichlorhydrate) : 170°C

### EXEMPLE 74 :

### 5-[4-(4-FLUOROBENZYL)PIPERAZIN-1-YL]-4H-PYRROLO[1,2-a]THIENO[3,2-e] PYRAZINE

Point de fusion (dichlorhydrate) : 180°C

### EXEMPLE 75 :

### 5-[2-(N,N-DIMETHYLAMINO)ETHYLAMINO]-4H-PYRROLO[1,2-a]THIENO[2,3-e] PYRAZINE

Point de fusion dichlorhydrate : 215°C

### EXEMPLE 76 :

### 5-[4-(3-PHENYLPROP-2-EN-1-YL)PIPERAZIN-1-YL]-4H-PYRROLO[1,2-a]THIENO[3,2-e]PYRAZINE

Point de fusion (dichlorhydrate) : 208°C

### EXEMPLE 77 :

### 5-{4-[2-(2-TRIFLUROMETHYLPHENYL)ETHYL]PIPERAZIN-1-YL}-4H-PYRROLO[1,2-a] THIENO[3,2-e]PYRAZINE

Point de fusion (dichlorhydrate) : 130°C

### EXEMPLE 78 :

### 5-{4-{2-[(METHYLAMINO)SULFONYL]ETHYL}PIPERAZIN-1-YL}-4H-PYRROLO[1,2-a] THIENO[3,2-e]PYRAZINE

### EXEMPLE 79 :

### ACIDE 3-[4-(4H-PYRROLO[1,2-a]THIENO[3,2-e]PYRAZIN-5-YL)PIPERAZIN-1-YL] PROPIONIQUE

### EXEMPLE 80 :

### 2-BENZYLOXY-5-(4-BENZYLPIPERAZIN-1-YL)-4H-PYRROLO[1,2-a]THIENO[3,2-e] PYRAZINE

### ETUDE PHARMACOLOGIQUE DES COMPOSES DE L'INVENTION

### EXEMPLE A :

### ETUDE DE LIAISON AUX RECEPTEURS DES COMPOSES DE L'INVENTION

### A-1 : Etude de liaison aux récepteurs sérotoninergiques 5-HT₃

La liaison des composés de l'invention aux récepteurs 5-HT₃ a été déterminée pour chaque composé par la mesure du déplacement du ³H zacopride (ligand spécifique des récepteurs 5-HT₃) dans des homogénats d'area postrema de rat.

### PROTOCOLE :

Des aliquotes (50-100 µl) d'une suspension membranaire (2,5-5,0 mg de protéines/cm³) sont mises à incuber pendant 30 min à 25°C dans un tampon Tris-HCl, 25 mM, p.H. 7.4, en présence de 0,3-0,4 nM de ³H zacopride (83 Ci/mmol) (volume total : 0,5 cm³). L'incubation est arrêtée par filtration des échantillons au travers de filtres Whatman GF/B qui sont ensuite lavés et séchés. La radioactivité retenue sur les filtres est finalement comptée par spectrométrie en milieu liquide (Aquasol). La liaison non spécifique est estimée dans les mêmes conditions à partir d'échantillons contenant en plus 1 µM d'ondansetron (antagoniste 5-HT₃).
Les courbes de déplacement obtenues en ajoutant des concentrations croissantes d'un composé à tester dans le milieu d'incubation sont analysées pour déterminer l'affinité des composés à tester pour les récepteurs 5-HT₃.

### A-2 : Etude de la liaison des composés de l'invention aux récepteurs sérotoninergiques 5-HT_{1A}, 5-HT_{1B}, 5-HT_{1C}, 5-HT_{1D} et 5-HT₂

La liaison des composés de l'invention (testés sur chaque récepteur à 6 concentrations différentes : de 10⁻⁴M à 10⁻⁹M) est mesurée :
- pour les récepteurs 5-HT_{1A}, par le déplacement de 8-OH-DPAT dans des homogénats d'hippocampe de rat,
- pour les récepteurs 5-HT_{1B}, par le déplacement de 5-hydroxytryptamine dans des homogénats de cortex, striatum et globus pallidus de rat,
- pour les récepteurs 5-HT_{1C}, par le déplacement de N-méthyl mésulergine dans des homogénats de cortex frontal et d'hippocampe de rat,
- pour les récepteurs 5-HT_{1D}, par le déplacement de 5-OH-tryptamine dans des homogénats de cortex, de striatum et de globus pallidus du rat,
- pour les récepteurs 5-HT₂, par le déplacement d'aminoiodo kétansérine dans des homogénats de cortex frontal de rat.

### CONCLUSION :

Il apparaît que les composés de l'invention possèdent une très haute affinité pour les récepteurs 5HT-3. Les composés de l'invention s'avèrent également posséder une haute sélectivité pour les récepteurs 5-HT₃ par rapport aux autres récepteurs sérotoninergiques.

### EXEMPLE B :

### MESURE DE L'ACCUMULATION DE 14C-GUANIDINIUM DANS LES CELLULES NG 108-15

L'accumulation de 14C-guanidinium dans les cellules NG 108-15 est mesurée et permet d'étudier l'interaction des composés testés avec les récepteurs 5-HT₃, sachant que cette accumulation est stimulée par les agonistes des récepteurs 5-HT₃.

Le clone hybride (neuroblastome-gliome) NG 108-15 est cultivé dans les conditions standards (milieu de Dulbecco auquel on ajoute 40 mM de bicarbonate de sodium, 1,8 mM de L-glutamine, 0,1 mM d'hypoxanthine, 1 µM d'aminoptérine, 16 µM de thymine et 10% de sérum de veau foetal) à 37°C dans une atmosphère enrichie en CO₂ (7%). A confluence (≃ 3,5 × 10⁵ cellules dans des boites de 35 mm de diamètre), le milieu de culture est aspiré et le tapis de cellules est lavé rapidement avec 3 cm³ d'un tampon HEPES (20 mM) contenant 145 mM de NaCl, 5,4 mM de KCl, 1,8 mM de CaCl2, 1,0 mM de MgCl2 et 20 mM de glucose, pH 7,4. Après élimination de la solution de lavage, on verse doucement dans chaque boîte de culture 1,5 cm³ du même tampon (excepté que la concentration de NaCl est ramenée à 135 mM) auquel on a ajouté 10 mM de chlorure de guanidinium plus 100-250 nCi de 14C-guanidinium, 10 µM de susbtance P, et un agoniste des récepteurs 5HT-3 en présence ou en absence du composé à tester. L'incubation dure 10 min à 37°C. Le milieu est ensuite aspiré, et les cellules sont rapidement lavées deux fois avec 3 cm³ du tampon de lavage dans lequel le NaCl a été intégralement remplacé par une concentration équimolaire de chlorure de choline. Finalement, le tapis de cellules est repris dans 0,5 cm³ de soude 0,4 N, et la radioactivité accumulée est comptée par spectrométrie en milieu liquide (Aquasol).

Dans ces conditions expérimentales, les agonistes des récepteurs 5-HT3 (10 µM - 0,1 mM) tels la sérotonine, la 2-méthyl-5-tryptamine, et le phénylbiguanide multiplient par 5 l'accumulation intracellulaire de 14C-guanidinium, et cet effet peut être complétement supprimé par 10-100 nM d'un antagoniste 5-HT3 sélectif (ondansetron, zacopride).

Ce test a permis de montrer que certains composés de l'invention sont de puissants antagonistes des récepteurs 5-HT3.

### EXEMPLE C :

### REFLEXE BRADYCARDIQUE DE BEZOLD-JARISCH

La bradycardie (réflexe de BEZOLD-JARISCH) est induite par l'injection intraveineuse d'agonistes 5-HT3 chez le rat anesthésié à l'uréthane.
Cette réponse consiste en une bradycardie brutale mais transitoire induite par la stimulation des récepteurs 5-HT3 sur les afférences vagales. Ce modèle est donc tout à fait approprié pour révéler d'éventuels effet agonistes ou antagonistes de composés qui sont des ligands potentiels des récepteurs 5-HT3.

### PROTOCOLE :

L'animal (rat mâle, adulte - souche Sprague-Dawley, 250-300 g) est tout d'abord anesthésié à l'uréthane (1,4 g/kg i.p.) puis trachéotomisé pour la mise en place d'une canule trachéale. Un cathéter (0,3 mm) est introduit dans l'aorte abdominale via l'artère fémorale pour l'enregistrement en continu de la pression artérielle et du rythme cardiaque. Les agents pharmacologiques sont injectés dans la veine saphène qui a été canulée à cet effet. L'injection i.v. de 30 µg de sérotonine, de 2-méthyl-5 hydroxytryptamine, ou de phénylbiguanide entraine une chute brutale (-80%) et transitoire (pendant environ 4-5 secondes) du rythme cardiaque, et cet effet peut être complétement empêché par l'administration (i.v.) préalable de divers antagonistes sélectifs tels le zacopride ou l'ondansetron (à la dose de 1 µg/kg).

### RESULTATS :

Il apparaît, dans ce test, que les composés de l'invention se comportent comme de puissants ligands des récepteurs 5-HT₃, et possédant une action soit antagoniste, soit agoniste.

### EXEMPLE E :

### COMPOSITION PHARMACEUTIQUE : COMPRIMES

Comprimés dosés à 5 mg de 5-(4-benzylpipérazin-1-yl)pyrrolo[1,2-a]thiéno[3,2-e]pyrazine.

Formule de préparation pour 1000 comprimés :

| | |
|---|---|
| 5-(4-benzylpipérazin-1-yl)pyrrolo[1,2-a]thiéno[3,2-e]pyrazine | 5 g |
| Amidon de blé | 2,5 g |
| Amidon de maïs | 1,5 g |
| Lactose | 8,5 g |
| Stéarate de magnésium | 0,2 g |
| Silice | 0,1 g |
| Hydroxypropylcellulose | 0,2 g |

### EXEMPLE F :

### COMPOSITION PHARMACEUTIQUE : COMPRIMES

Comprimés dosés à 1 mg de 5-(4-benzylpipérazin-1-yl)pyrrolo[1,2-a]thiéno[3,2-e]pyrazine.

Formule de préparation pour 1000 comprimés :

| | |
|---|---|
| 5-(4-benzylpipérazin-1-yl)pyrrolo[1,2-a]thiéno[3,2-e]pyrazine | 1 g |
| Amidon de blé | 2,5 g |
| Amidon de maïs | 1,5 g |
| Lactose | 8,5 g |
| Stéarate de magnésium | 0,2 g |
| Silice | 0,1 g |
| Hydroxypropylcellulose | 0,2 g |

## Revendications

1. Composés de formule (I) : 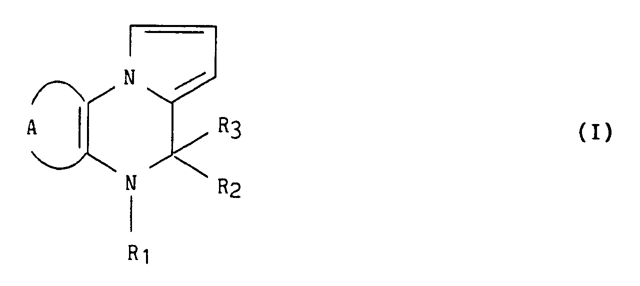 dans laquelle :
- soit R₁ et R₂ forment ensemble une double liaison et R₃ représente un atome de chlore ou un groupement de formule : 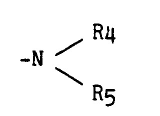 dans laquelle R₄ et R₅ forment avec l'atome d'azote qui les porte un groupement choisi parmi :
- pipérazine,
- pipérazine substituée,
- pipéridine substituée,
- pyrrolidine substituée,
- morpholine substituée par un ou plusieurs radicaux alkyles,
- tétrahydropyridine,
- thiomorpholine,
- azaspirane de 5 à 12 chaînons,
- azaspirane de 5 à 12 chaînons substitué par un ou plusieurs radicaux alkyles ou groupements oxo,
- azacycloalkyle de 7 à 12 chaînons incluant éventuellement dans son squelette de 1 à 2 hétéroatomes supplémentaires choisis parmi oxygène, soufre, et azote,
- azacycloalkyle de 7 à 12 chaînons substitué par un ou plusieurs radicaux alkyles ou groupements oxo incluant éventuellement dans son squelette de 1 à 2 hétéroatomes supplémentaires choisis parmi oxygène, soufre, et azote,
- un groupement -NH-(CH₂)ₖ-NH₂ dans lequel k représente un nombre entier égal à 2, 3, ou 4,
- et un groupement -NH(CH₂)ₖ-NH₂ substitué dans lequel k représente un nombre entier égal à 2, 3, ou 4,
- soit R₁ représente un atome d'hydrogène, et R₂ et R₃ forment ensemble un groupement oxo,
étant entendu que le terme "substitué(e)" affectant ci-dessus les groupements pipérazine, pipéridine, pyrrolidine, et -NH-(CH₂)ₖ-NH₂ signifie que ces groupements peuvent être substitués par un ou plusieurs atomes d'halogène, radicaux hydroxyles, radicaux oxo, radicaux R₁₀, ou radicaux 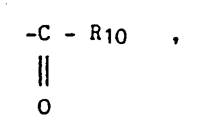 avec R₁₀ étant choisi parmi :
- alkyle,
- alkoxy,
- alcényle,
- -(CH₂)ₙ-R₁₁ ou 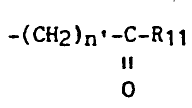 où n représente 0 ou un nombre entier de 1 à 5, n' représente un nombre entier de 1 à 5, et où R₁₁ représente un radical phényle, benzhydryle, thiényle, pyrrolyle, pyrrolidinyle, furyle, pyrimidinyle, pyridyle, benzodioxolyle, benzodioxanyle, naphtyle, quinoléinyle, isoquinoléinyle, cycloalkyle, et dicycloalkylméthyle, le terme cycloalkyle représentant un groupement mono- ou bi-cyclique, de 3 à 12 chaînons,
ces radicaux R₁₁ pouvant eux-mêmes être substitués par un ou plusieurs radicaux choisis parmi halogène, trifluorométhyle, amino, nitro, hydroxyle, alkyle, et alkoxy,
- et -(CH₂)_{n'}-R₁₂ où n' est tel que défini précédemment et R₁₂ représente un groupement choisi parmi carboxyle, alkoxycarbonyle, amino, dialkylamino, -SO₂NR₁₃R₁₄, et -CONR₁₃R₁₄ is dans lesquels R₁₃ et R₁₄ représentent chacun, indépendamment l'un de l'autre, un atome d'hydrogène ou un alkyle,
. et A signifie :
- soit un groupement de formule (α) : 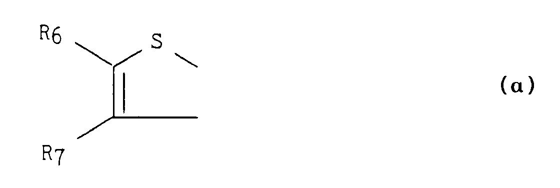 et forme avec le système hétérocyclique qui le porte une pyrrolo[1,2-a] thiéno[3,2-e]pyrazine de formule (I/α) : 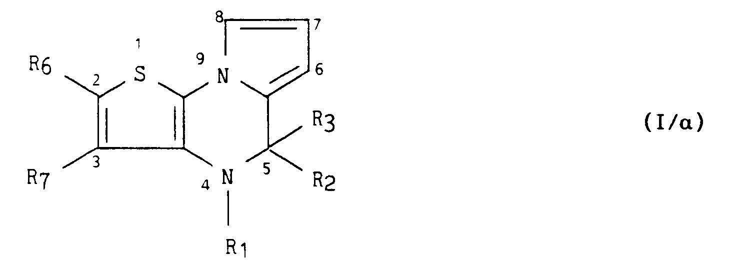 dans laquelle R₁, R₂, et R₃ sont tels que définis précédemment et R₆ et R₇, identiques ou différents, représentent chacun indépendamment l'un de l'autre un groupement choisi parmi :
- hydrogène,
- alkyle,
- alkyle substitué par un ou plusieurs atomes d'halogène ou radicaux hydroxyle,
- acyle,
- alkoxycarbonyle,
- halogène,
- nitro,
- -(CH₂)ₘ-phényle ou -(CH₂)ₘ-napthyle, ou -(CH₂)ₘ-pyridyle, où m représente 0 ou un nombre entier de 1 à 4 et où le noyau phényle est non substitué ou substitué par un ou plusieurs radicaux Ra, identiques ou différents, avec Ra signifiant halogène, alkyle, alkoxy, trifluorométhyle, ou pyrrolidinyle,
- et un groupe de formule : 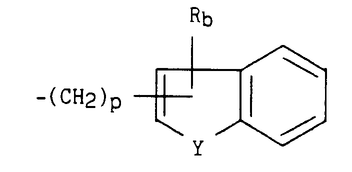 dans laquelle p représente 0 ou un nombre entier de 1 à 2, Y représente un atome d'oxygène, d'azote, ou de soufre et R_{b} représente un atome d'hydrogène ou un groupement alkyle,
- soit un groupement de formule (β) : 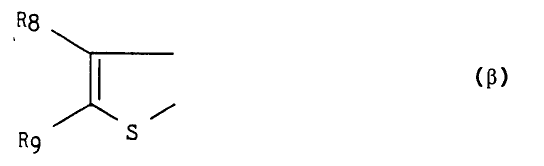 et forme avec le système hétérocyclique qui le porte une pyrrolo [1,2-a] thiéno[2,3-e]pyrazine de formule (I/β) : 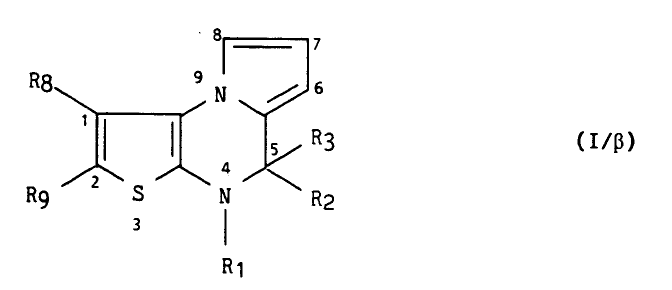 dans laquelle R₁, R₂, et R₃ sont tels que définis précédemment et R₈ et R₉ ont les mêmes significations que R₆ et R₇ tels que définis précédemment,
étant entendu que si R₃ représente un atome de chlore ou si R₃ et R₂ forment ensemble un groupement oxo, alors R₆ et R₇, ou R₈ et R₉, ne peuvent pas représenter simultanément deux atomes d'hydrogène,
leurs isomères optiques, sous forme pure ou sous forme de mélange, quand R₃, R₆, R₇, R₈ ou R₉ représente un groupement optiquement actif,
et leurs sels d'addition à un acide ou à une base pharmaceutiquement acceptable,
étant entendu que, sauf précisions contraires,
- les termes "alkyle", "alkoxy", et "acyle" représentent des groupements linéaires ou ramifiés possédant de 1 à 6 atomes de carbone,
- et le terme "alcényle" représente un groupement insaturé, linéaire ou ramifié, possédant de 2 à 6 atomes de carbone.

2. Composés de formule (I) selon la revendication 1 dans laquelle R1 et R₂ forment ensemble une double liaison et R3 représente un groupement de formule : 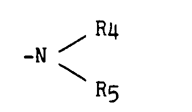 où R₄ et R₅ sont tels que définis dans la revendication 1,
leurs isomères optiques,
et leurs sels d'addition à un acide ou à une base pharmaceutiquement acceptable.

3. Composés de formule (I) selon la revendication 1 dans laquelle R₁ et R₂ forment ensemble une double liaison et R₃ représente un groupement de formule : 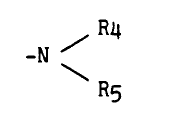 où R₄ et R₅ forment avec l'atome d'azote qui les porte une pipérazine ou une pipérazine substituée, le terme "substituée" étant tel que défini dans la revendication 1,
leurs isomères optiques,
et leurs sels d'addition à un acide ou à une base pharmaceutiquement acceptable.

4. Composé de formule (I) selon la revendication 1 qui est la 5-(4-benzylpipérazin-1-yl)pyrrolo[1,2-a]thiéno[3,2-e]pyrazine et ses sels d'addition à un acide pharmaceutiquement acceptable.

5. Composé de formule (I) selon la revendication 1 qui est la 5-(pipérazin-1-yl)pyrrolo[1,2-a]thiéno[3,2-e]pyrazine et ses sels d'addition à un acide pharmaceutiquement acceptable.

6. Composé de formule (I) selon la revendication 1 qui est la 5-(4-méthylpipérazin-1-yl)pyrrolo[1,2-a]thiéno[3,2-e]pyrazine et ses sels d'addition à un acide pharmaceutiquement acceptable.

7. Procédé de préparation des composés de formule (I) selon la revendication 1 caractérisé en ce que : on chauffe, en présence ou en absence de solvant, un composé de formule (II), 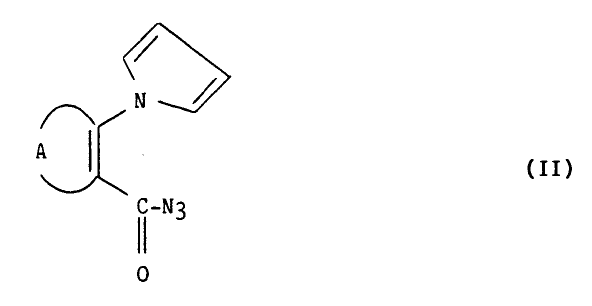 dans laquelle A est tel que défini dans la formule (I) selon la revendication 1,
pour obtenir un composé de formule (I/a) : 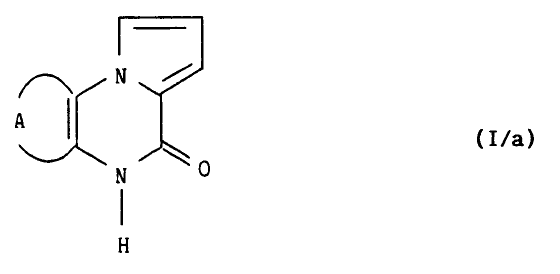 dans laquelle A est tel que défini précédemment,
cas particulier des composés de formule (I) selon la revendication 1 dans lesquels R₁ représente un atome d'hydrogène et R₂ et R₃ forment ensemble un groupement oxo,
composé de formule (I/a) qui est soumis à un agent d'halogénation pour obtenir un composé de formule (I/b) : 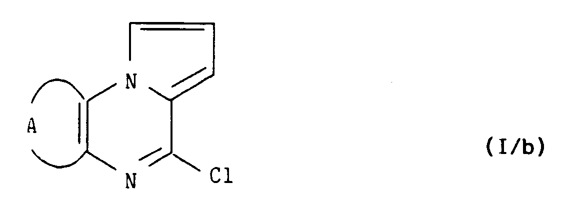 dans laquelle A est tel que défini précédemment,
cas particulier des composés de formule (I) selon la revendication 1 dans lesquels R₁ et R₂ forment ensemble une double liaison et R₃ représente un atome de chlore,
qui est ensuite mis en réaction avec une amine de formule (III) : 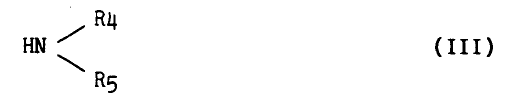 dans laquelle R₄ et R₅ sont tels que définis dans la formule (I) selon la revendication 1,
pour obtenir un composé de formule (I/c) : 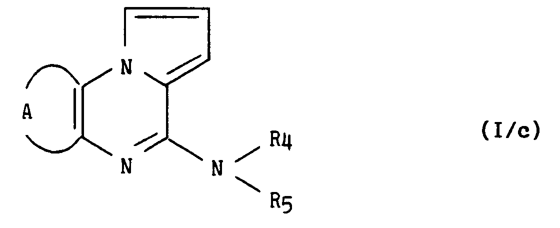 dans laquelle A, R₄, et R₅ sont tels que définis précédemment,
cas particulier des composés de formule (I) selon la revendication 1 dans lesquels R₁ et R₂ forment une double liaison et R₃ représente un groupement : 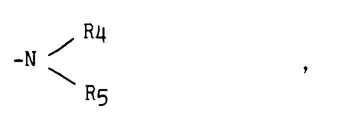 les composés de formule (I/a), (I/b), (I/c) formant l'ensemble des composés de formule (I) selon la revendication 1, les composés de formule (I) selon la revendication 1 pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- et/ou salifiés par un acide ou par une base pharmaceutiquement acceptable.

8. Procédé de préparation des composés de formule (I/c), cas particulier des composés de formule (I) selon la revendication 1 dans lesquels R₁ et R₂ forment ensemble une double liaison et R₃ représente un groupement : 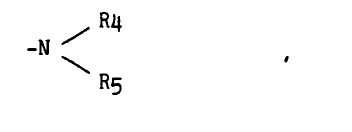 caractérisé en ce que :
on fait réagir un composé de formule (II) : 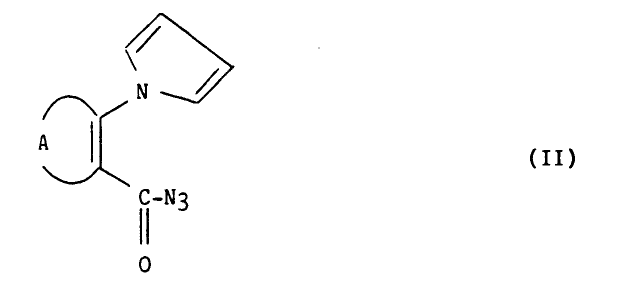 dans laquelle A est tel que défini dans la formule (I) selon la revendication 1,
avec une amine de formule (III) : 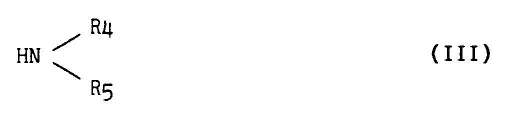 dans laquelle R₄ et R₅ sont tels que définis dans la formule (I) selon la revendication 1 pour obtenir un composé de formule (V) : 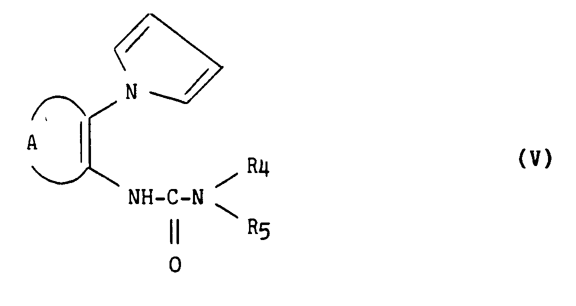 dans laquelle A, R₄ et R₅ sont tels que définis précédemment,
qui est ensuite cyclisé sous l'action d'un agent d'halogénation et soumis à un traitement alcalin pour donner un composé de formule (I/c) : 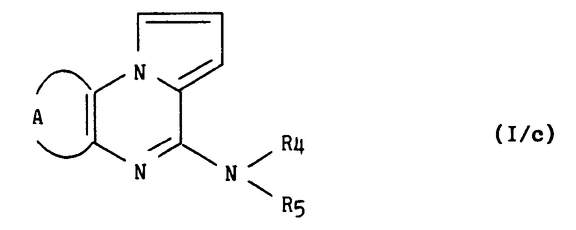 dans laquelle A, R₄, et R₅ sont tels que définis précédemment,
cas particulier des composés de formule (I) selon la revendication 1 dans lesquels R₁ et R₂ forment ensemble une double liaison et R₃ représente un groupement : 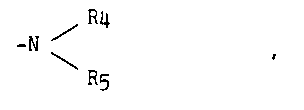 les composés de formule (I/c) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères optiques,
- et/ou salifiés par un acide ou par une base pharmaceutiquement acceptable.

9. Composition pharmaceutique contenant comme principe actif au moins un des composés de formule (I) selon la revendication 1 ou un de ses sels d'addition avec un acide ou avec une base pharmaceutiquement acceptable en combinaison avec un ou plusieurs excipients ou véhicules pharmaceutiquement acceptables.

10. Composition pharmaceutique selon la revendication 9 utile dans la prévention et le traitement de l'anxiété, de la dépression, du stress, des psychoses, de la schizophrénie, des troubles du système nerveux central, de la migraine, des troubles de la mémoire, des troubles du comportement alimentaire, de l'alcoolisme, de la douleur, ainsi que dans la prévention et le traitement du vomissement et des désordres de la vidange gastrique.

## Claims

1. Compounds of formula (I): 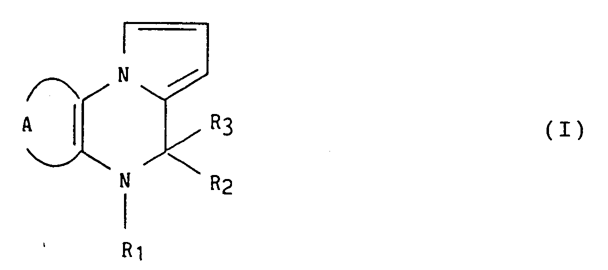 in which:
- either R₁ and R₂ together form a double bond and R₃ represents a chlorine atom or a group of the formula: 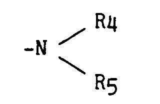 in which R₄ and R₅, with the nitrogen atom carrying them, form a group selected from:
- piperazine,
- substituted piperazine,
- substituted piperidine,
- substituted pyrrolidine,
- morpholine substituted by one or more alkyl radicals,
- tetrahydropyridine,
- thiomorpholine,
- azaspirane having from 5 to 12 ring members,
- azaspirane having from 5 to 12 ring members that is substituted by one or more alkyl radicals or oxo groups,
- azacycloalkyl having from 7 to 12 ring members that optionally includes in its skeleton 1 or 2 additional hetero atoms selected from oxygen, sulphur and nitrogen,
- azacycloalkyl having from 7 to 12 ring members that is substituted by one or more alkyl radicals or oxo groups and that optionally includes in its skeleton 1 or 2 additional hetero atoms selected from oxygen, sulphur and nitrogen,
- a -NH-(CH₂)ₖ-NH₂ group in which k represents an integer 2, 3 or 4,
- and a substituted -NH-(CH₂)ₖ-NH₂ group in which k represents an integer 2, 3 or 4,
- or R₁ represents a hydrogen atom and R₂ and R₃ together form an oxo group,
wherein the term "substituted" relating above to the groups piperazine, piperidine, pyrrolidine and -NH-(CH₂)ₖ-NH₂ indicates that those groups may be substituted by one or more halogen atoms, hydroxy radicals, oxo radicals, radicals R₁₀ or radicals 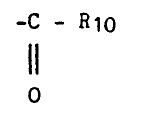 R₁₀ being selected from:
- alkyl,
- alkoxy,
- alkenyl,
- -(CH₂)ₙ-R₁₁ or 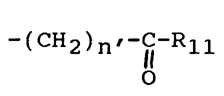 wherein n represents 0 or an integer from 1 to 5, n' represents an integer from 1 to 5, and R₁₁ represents a phenyl, benzhydryl, thienyl, pyrrolyl, pyrrolidinyl, furyl, pyrimidinyl, pyridyl, benzodioxolyl, benzodioxanyl, naphthyl, quinolinyl, isoquinolinyl, cycloalkyl or dicycloalkylmethyl radical, the term cycloalkyl representing a mono- or bi-cyclic group having from 3 to 12 ring members,
it being possible for those radicals R₁₁ themselves to be substituted by one or more radicals selected from halogen, trifluoromethyl, amino, nitro, hydroxy, alkyl and alkoxy,
- and -(CH₂)_{n'}-R₁₂ wherein n' is as defined above and R₁₂ represents a group selected from carboxy, alkoxycarbonyl, amino, dialkylamino, -SO₂NR₁₃R₁₄ and -CONR₁₃R₁₄ in which each of R₁₃ and R₁₄ independently of the other represents a hydrogen atom or an alkyl radical,
. and A represents:
- either a group of formula (α): 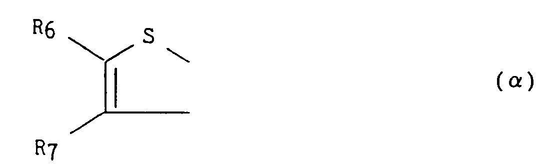 and forms, with the heterocyclic system carrying it, a pyrrolo[1,2-a]thieno[3,2-e]pyrazine of formula (I/α): 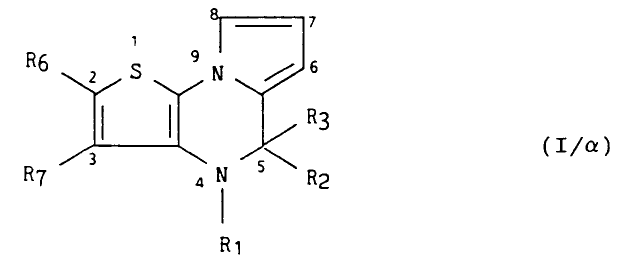 in which R₁, R₂ and R₃ are as defined above and each of R₆ and R₇, which may be identical or different, independently of the other represents a group selected from:
- hydrogen,
- alkyl,
- alkyl substituted by one or more halogen atoms or hydroxy radicals,
- acyl,
- alkoxycarbonyl,
- halogen,
- nitro,
- -(CH₂)ₘ-phenyl, -(CH₂)ₘ-naphthyl or -(CH₂)ₘ-pyridyl wherein m represents 0 or an integer from 1 to 4 and wherein the phenyl nucleus is unsubstituted or substituted by one or more radicals Ra, which may be identical or different, Ra representing halogen, alkyl, alkoxy, trifluoromethyl or pyrrolidinyl,
- and a group of the formula: 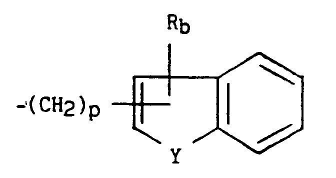 in which p represents 0 or an integer 1 or 2, Y represents an oxygen atom, a nitrogen atom or a sulphur atom, and R_{b} represents a hydrogen atom or an alkyl group,
- or a group of formula (β): 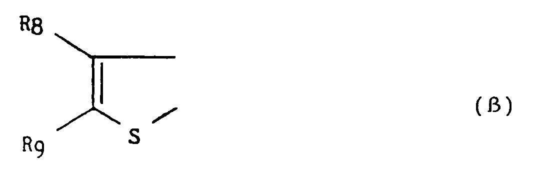 and forms, with the heterocyclic system carrying it, a pyrrolo[1,2-a]thieno[2,3-e]pyrazine of formula (I/β): 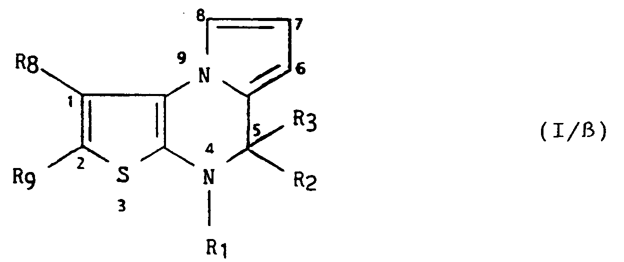 in which R₁, R₂ and R₃ are as defined above and R₈ and R₉ have the same meanings as R₆ and R₇ as defined above, with the proviso that when R₃ represents a chlorine atom, or when R₃ and R₂ together form an oxo group, R₆ and R₇, or R₈ and R₉, may not at the same time be two hydrogen atoms,
their optical isomers, in pure form or in the form of a mixture, when R₃, R₆, R₇, R₈ or R₉ represents an optically active group,
and addition salts thereof with a pharmaceutically acceptable acid or base,
wherein, unless indicated otherwise,
- the terms "alkyl", "alkoxy" and "acyl" represent linear or branched groups having from 1 to 6 carbon atoms,
- and the term "alkenyl" represents an unsaturated, linear or branched group having from 2 to 6 carbon atoms.

2. Compounds of formula (I) according to claim 1 in which R₁ and R₂ together form a double bond and R₃ represents a group of the formula: 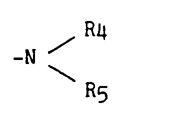 wherein R₄ and R₅ are as defined in claim 1,
their optical isomers, and addition salts thereof with a pharmaceutically accep
table acid or base.

3. Compounds of formula (I) according to claim 1 in which R₁ and R₂ together form a double bond and R₃ represents a group of the formula: 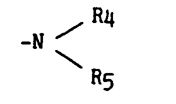 wherein R₄ and R₅, with the nitrogen atom carrying them, form a piperazine group or a substituted piperazine group, the term "substituted" being as defined in claim 1,
their optical isomers,
and addition salts thereof with a pharmaceutically acceptable acid or base.

4. A compound of formula (I) according to claim 1 which is 5-(4-benzylpiperazin-1-yl)pyrrolo[1,2-a]thieno[3,2-e]pyrazine, and its addition salts with a pharmaceutically acceptable acid.

5. A compound of formula (I) according to claim 1 which is 5-(piperazin-1-yl)pyrrolo[1,2-a]thieno[3,2-e]pyrazine, and its addition salts with a pharmaceutically acceptable acid.

6. A compound of formula (I) according to claim 1 which is 5-(4-methylpiperazin-1-yl)pyrrolo[1,2-a]thieno[3,2-e]pyrazine, and its addition salts with a pharmaceutically acceptable acid.

7. Process for the preparation of compounds of formula (I) according to claim 1, characterised in that: a compound of formula (II): 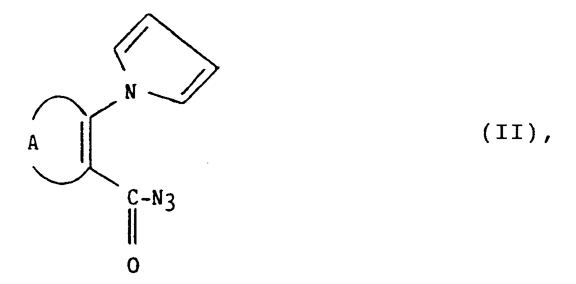 in which A is as defined in formula (I) according to claim 1,
is heated, in the presence or in the absence of a solvent, to give a compound of formula (I/a): 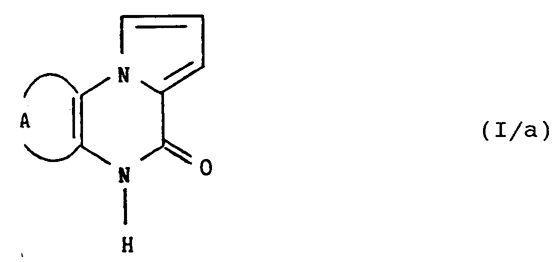 in which A is as defined above,
which is a particular case of the compounds of formula (I) according to claim 1 in which R₁ represents a hydrogen atom and R₂ and R₃ together form an oxo group,
which compound of formula (I/a) is subjected to a halogenating agent to give a compound of formula (I/b): 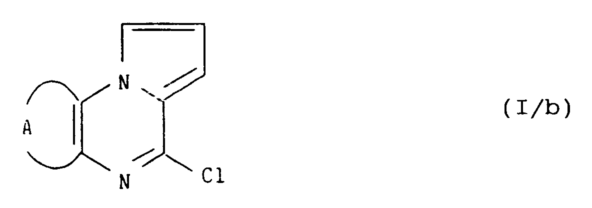 in which A is as defined above,
which is a particular case of the compounds of formula (I) according to claim 1 in which R₁ and R₂ together form a double bond and R₃ represents a chlorine atom,
which is then reacted with an amine of formula (III): 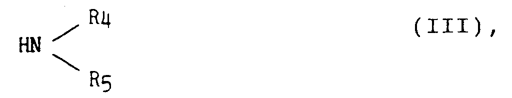 in which R₄ and R₅ are as defined in formula (I) according to claim 1,
to give a compound of formula (I/c): 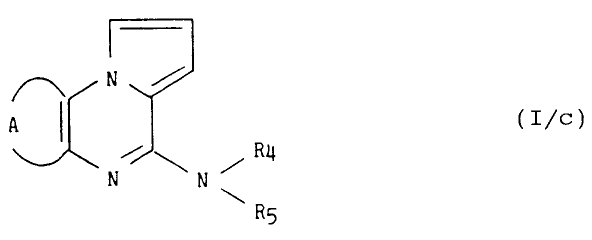 in which A, R₄ and R₅ are as defined above,
which is a particular case of the compounds of formula (I) according to claim 1 in which R₁ and R₂ form a double bond and R₃ represents a group: 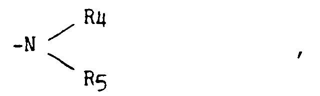 the compounds of formulae (I/a), (I/b) and (I/c) forming the compounds of formula (I) according to claim 1 in their entirety,
it being possible for the compounds of formula (I) according to claim 1 to be:
- purified by one or more purification methods selected from crystallisation, chromatography over a silica column, extraction, filtration, and passage over charcoal or resin,
- separated, where applicable, in pure form or in the form of a mixture, into their possible optical isomers,
- and/or converted into a salt with a pharmaceutically acceptable acid or base.

8. Process for the preparation of compounds of formula (I/c), which are a particular case of the compounds of formula (I) according to claim 1 in which R₁ and R₂ together form a double bond and R₃ represents a group: 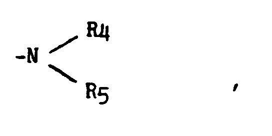 characterised in that:
a compound of formula (II): 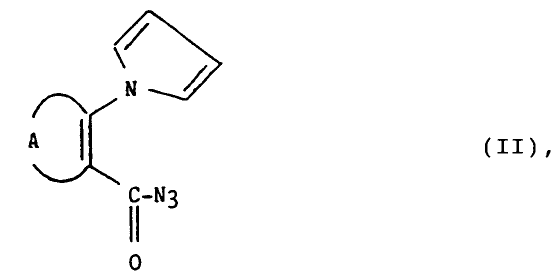 in which A is as defined in formula (I) according to claim 1,
is reacted with an amine of formula (III): 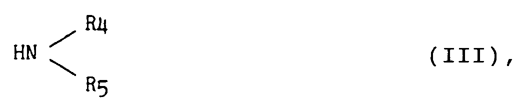 in which R₄ and R₅ are as defined in formula (I) according to claim 1,
to give a compound of formula (V): 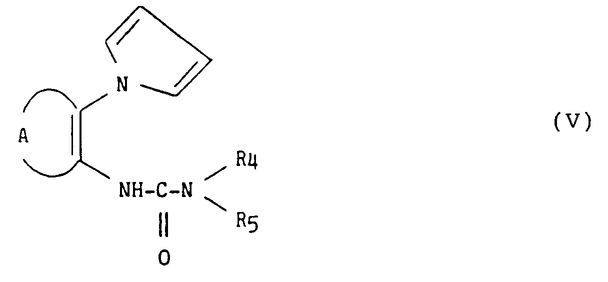 in which A, R₄ and R₅ are as defined above,
which is then cyclised under the action of a halogenating agent and subjected to alkaline treatment to give a compound of formula (I/c): 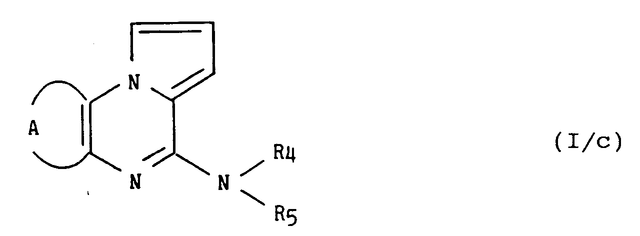 in which A, R₄ and R₅ are as defined above,
which is a particular case of the compounds of formula (I) according to claim 1 in which R₁ and R₂ together form a double bond and R₃ represents a group: 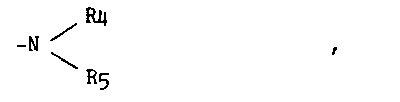 it being possible for the compounds of formula (I/c) to be:
- purified by one or more purification methods selected from crystallisation, chromatography over a silica column, extraction, filtration, and passage over charcoal or resin,
- separated, where applicable, in pure form or in the form of a mixture, into their possible optical isomers,
- and/or converted into a salt with a pharmaceutically acceptable acid or base.

9. Pharmaceutical composition comprising as active ingredient at least one of the compounds of formula (I) according to claim 1 or an addition salt thereof with a pharmaceutically acceptable acid or base, in combination with one or more pharmaceutically acceptable excipients or carriers.

10. Pharmaceutical composition according to claim 9 for use in the prevention and treatment of anxiety, depression, stress, psychoses, schizophrenia, disorders of the central nervous system, migraine, memory disorders, eating disorders, alcoholism and pain, as well as in the prevention and treatment of vomiting and gastric emptying disorders.

## Patentansprüche

1. Verbindungen der Formel (I): 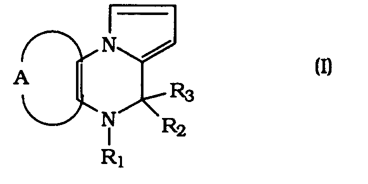 in der:
- entweder R₁ und R₂ gemeinsam eine Doppelbindung bilden und R₃ ein Chloratom oder eine Gruppe der Formel: 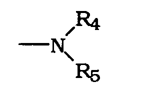 darstellt, in der R₄ und R₅ zusammen mit dem sie tragenden Stickstoffatom eine Gruppe bilden ausgewählt aus:
- Piperazin,
- substituiertem Piperazin,
- substituiertem Piperidin,
- substituiertem Pyrrolidin,
- durch eine oder mehrere Alkylgruppen substituiertem Morpholin,
- Tetrahydropyridin,
- Thiomorpholin,
- Azaspiran mit 5 bis 12 Kettengliedern,
- durch eine oder mehrere Alkylgruppen oder Oxogruppen substituiertem Azaspiran mit 5 bis 12 Kettengliedern,
- Azacycloalkyl mit 7 bis 12 Kettengliedern, welches gegebenenfalls in seinem Gerüst 1 bis 2 zusätzliche Heteroatome ausgewählt aus Sauerstoff, Schwefel und Stickstoff aufweist,
- durch eine oder mehrere Alkylgruppen oder Oxogruppen substituiertem Azacycloalkyl mit 7 bis 12 Kettengliedern, welches gegebenenfalls in seinem Gerüst 1 bis 2 zusätzliche Heteroatome ausgewählt aus Sauerstoff, Schwefel und Stickstoff aufweist,
- einer Gruppe -NH-(CH₂)ₖ-NH₂, in der k eine ganze Zahl mit einem Wert von 2, 3 oder 4 darstellt, und
- einer substituierten Gruppe -NH-(CH₂)ₖ-NH₂, in der k eine ganze Zahl mit einem Wert von 2, 3 oder 4 darstellt,
bilden,
- oder R₁ ein Wasserstoffatom darstellt und R₂ und R₃ gemeinsam eine Oxogruppe bilden,
wobei es sich versteht, daß der oben benutzte Begriff "substituiert", wie er für die Piperazin-, Piperidin-, Pyrrolidingruppen und die Gruppe -NH-(CH₂)ₖ-NH₂ benutzt wird, bedeutet, daß diese Gruppen durch ein oder mehrere Halogenatome, Hydroxylgruppen, Oxogruppen, R₁₀-Gruppen oder Gruppen 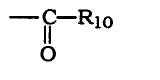 substituiert sein können,
wobei R₁₀ ausgewählt ist aus:
- Alkyl,
- Alkoxy,
- Alkenyl,
- -(CH₂)ₙ-R₁₁ oder 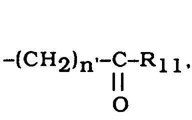 worin n 0 oder eine ganze Zahl mit einem Wert von 1 bis 5, n' eine ganze Zahl mit einem Wert von 1 bis 5 und R₁₁ eine Phenyl-, Benzhydryl-, Thienyl-, Pyrrolyl-, Pyrrolidinyl-, Furyl-, Pyrimidinyl-, Pyridyl-, Benzodioxolyl-, Benzodioxanyl-, Naphthyl-, Chinolinyl-, Isochinolinyl-, Cycloalkyl- oder Dicycloalkylmethylgruppe bedeuten, wobei der Begriff Cycloalkyl für eine mono- oder bicyclische Gruppe mit 3 bis 12 Kettengliedern steht
und die Gruppen R₁₁ ihrerseits durch eine oder mehrere Gruppen ausgewählt aus Halogenatomen, Trifluormethylgruppen, Aminogruppen, Nitrogruppen, Hydroxylgruppen, Alkylgruppen oder Alkoxygruppen substituiert sein können,
- und -(CH₂)_{n'}-R₁₂, in der n' die oben angegebenen Bedeutungen besitzt und R₁₂ eine Gruppe ausgewählt aus Carboxyl, Alkoxycarbonyl, Amino, Dialkylamino, -SO₂NR₁₃R₁₄ und -CONR₁₃R₁₄ darstellt, worin R₁₃ und R₁₄ jeweils unabhängig voneinander ein Wasserstoffatom oder eine Alkylgruppe bedeuten,
• und A:
- entweder eine Gruppe der Formel (α) darstellt: 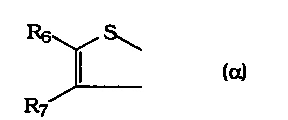 und zusammen mit dem sie tragenden heterocyclischen System ein Pyrrolo[1,2-a]thieno[3,2-e]pyrazin der Formel (I/α) bildet: 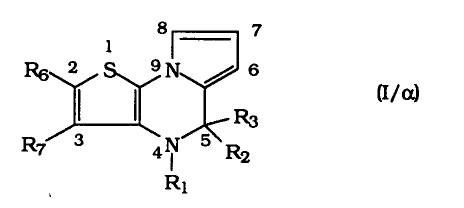 in der R₁, R₂ und R₃ die oben angegebenen Bedeutungen besitzen und R₆ und R₇, die gleichartig oder voneinander verschieden sein können, ausgewählt sind aus:
- Wasserstoff,
- Alkyl,
- durch ein oder mehrere Halogenatome oder Hydroxylgruppen substituiertem Alkyl,
- Acyl,
- Alkoxycarbonyl,
- Halogen,
- Nitro,
- -(CH₂)ₘ-Phenyl oder -(CH₂)ₘ-Naphthyl oder -(CH₂)ₘ-Pyridyl, worin m 0 oder eine ganze Zahl mit einem Wert von 1 bis 4 darstellt und der Phenylkern entweder unsubstituiert oder durch einen oder mehrere gleichartige oder verschiedene Reste Rₐ substituiert ist, wobei Rₐ für Halogen, Alkyl, Alkoxy, Trifluormethyl oder Pyrrolidinyl steht,
- und einer Gruppe der Formel: 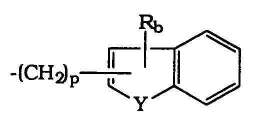 in der p 0 oder eine ganze Zahl mit einem Wert von 1 bis 2, Y ein Sauerstoffatom, ein Stickstoffatom oder ein Schwefelatom und R_{b} ein Wasserstoffatom oder eine Alkylgruppe bedeuten,
- oder eine Gruppe der Formel (β) darstellt: 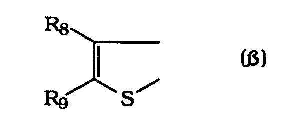 und mit dem sie tragenden heterocyclischen System ein Pyrrolo[1,2-a]thieno[2,3-e]pyrazin der Formel (I/β) bildet: 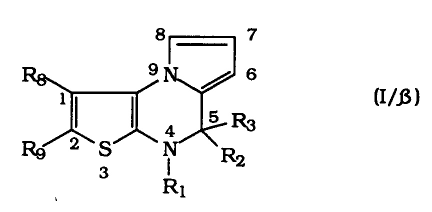 in der R₁, R₂ und R₃ die oben angegebenen Bedeutungen besitzen und R₈ und R₉ die gleichen Bedeutungen besitzen, wie sie oben für R₆ und R₇ definiert worden sind,
wobei es sich versteht, daß, wenn R₃ ein Chloratom bedeutet oder wenn R₃ und R₂ gemeinsam eine Oxogruppe bilden, dann R₆ und R₇ oder R₈ und R₉ nicht gleichzeitig zwei Wasserstoffatome bedeuten können,
deren optische Isomere in reiner Form oder in Form einer Mischung, wenn R₃, R₆, R₇, R₈ oder R₉ eine optisch aktive Gruppe bedeutet,
und deren Additionssalze mit einer pharmazeutisch annehmbaren Säure oder Base,
wobei, wenn nichts anderes angegeben ist,
- die Begriffe "Alkyl", "Alkoxy" und "Acyl" geradkettige oder verzweigte Gruppen mit 1 bis 6 Kohlenstoffatomen bedeuten,
- und der Begriff "Alkenyl" für eine geradkettige oder verzweigte ungesättigte Gruppe mit 2 bis 6 Kohlenstoffatomen steht.

2. Verbindungen der Formel (I) nach Anspruch 1, in der R₁ und R₂ gemeinsam eine Doppelbindung bilden und R₃ eine Gruppe der Formel bedeutet: 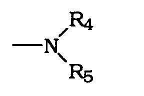 in der R₄ und R₅ die in Anspruch 1 angegebenen Bedeutungen besitzen,
deren optische Isomere und deren Additionssalze mit einer pharmazeutisch annehmbaren Säure oder Base.

3. Verbindungen der Formel (I) nach Anspruch 1, in der R₁ und R₂ gemeinsam eine Doppelbindung bilden und R₃ eine Gruppe der Formel bedeutet: 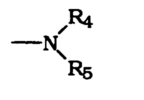 in der R₄ und R₅ gemeinsam mit dem sie tragenden Stickstoffatom einen Piperazin- oder substituierten Piperazinrest bilden, wobei der Begriff "substituiert" die in Anspruch 1 angegebenen Bedeutungen besitzt,
deren optische Isomeren und deren Additionssalze mit einer pharmazeutisch annehmbaren Säure oder Base.

4. Verbindung der Formel (I) nach Anspruch 1, nämlich 5-(4-Benzylpiperazin-1-yl)-pyrrolo[1,2-a]thieno[3,2-e]pyrazin und dessen Additionssalze mit einer pharmazeutisch annehmbaren Säure.

5. Verbindung der Formel (I) nach Anspruch 1, nämlich 5-(Piperazin-1-yl)-pyrrolo[1,2-a]thieno[3,2-e]pyrazin und dessen Additionssalze mit einer pharmazeutisch annehmbaren Säure.

6. Verbindung der Formel (I) nach Anspruch 1, nämlich 5-(4-Methylpiperazin-1-yl)-pyrrolo[1,2-a]thieno[3,2-e]pyrazin und dessen Additionssalze mit einer pharmazeutisch annehmbaren Säure.

7. Verfahren zur Herstellung der Verbindungen der Formel (I) nach Anspruch 1, dadurch gekennzeichnet, daß man:
eine Verbindung der Formel (II): 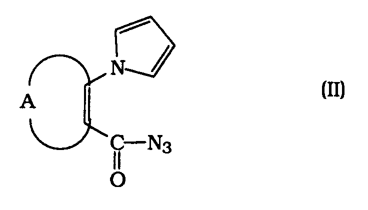 in der A die bezüglich der Formel (I) in Anspruch 1 angegebenen Bedeutungen besitzt, in Gegenwart oder in Abwesenheit eines Lösungsmittels erhitzt zur Bildung einer Verbindung der Formel (I/a): 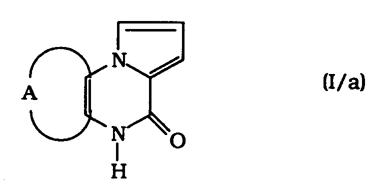 in der A die oben angegebenen Bedeutungen besitzt,
einem Sonderfall der Verbindungen der Formel (I) nach Anspruch 1, in der R₁ ein Wasserstoffatom und R₂ und R₃ gemeinsam eine Oxogruppe bedeuten,
welche Verbindung der Formel (I/a) der Einwirkung eines Halogenierungsmittels unterworfen wird zur Bildung einer Verbindung der Formel (I/b): 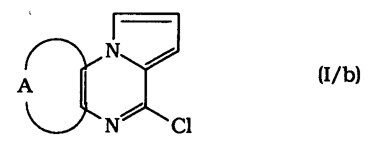 in der A die oben angegebenen Bedeutungen besitzt,
einem Sonderfall der Verbindungen der Formel (I) nach Anspruch 1, in der R₁ und R₂ gemeinsam eine Doppelbindung und R₃ ein Chloratom bedeuten,
welche anschließend mit einem Amin der Formel (III) umgesetzt wird: 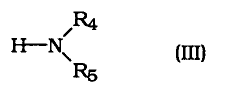 in der R₄ und R₅ die bezüglich der Formel (I) nach Anspruch 1 angegebenen Bedeutungen besitzen,
zur Bildung einer Verbindung der Formel (I/c): 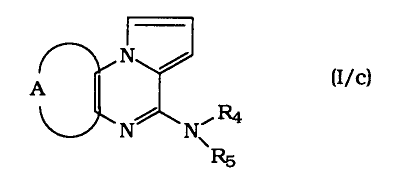 in der A, R₄ und R₅ die oben angegebenen Bedeutungen besitzen,
einem Sonderfall der Verbindungen der Formel (I) nach Anspruch 1, in der R₁ und R₂ eine Doppelbindung und R₃ eine Gruppe der Formel bedeuten: 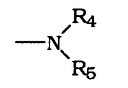 welche Verbindungen der Formeln (I/a), (I/b) und (I/c) die Gesamtheit der Verbindungen der Formel (I) nach Anspruch 1 bilden,
welche Verbindungen der Formel (I) nach Anspruch 1:
- nach einer oder mehreren Reinigungsmethoden ausgewählt aus Kristallisation, Säulenchromatographie über Siliciumdioxid, Extraktion, Filtration und Überführung über Kohlenstoff oder ein Harz gereinigt werden können,
- gegebenenfalls in ihre eventuellen optischen Isomeren in reiner Form oder in Form einer Mischung getrennt werden können,
- und/oder durch Einwirkung einer pharmazeutisch annehmbaren Säure oder Base in die Salze überführt werden können.

8. Verfahren zur Herstellung der Verbindungen der Formel (I/c), einem Sonderfall der Verbindungen der Formel (I) nach Anspruch 1, in der R₁ und R₂ gemeinsam eine Doppelbindung und R₃ eine Gruppe der Formel bedeuten: 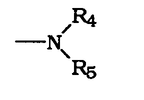 dadurch gekennzeichnet, daß man:
eine Verbindung der Formel (II): 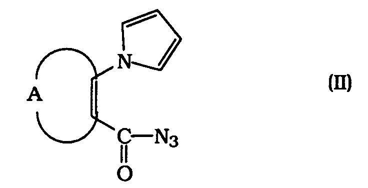 in der A die bezüglich der Formel (I) in Anspruch 1 angegebenen Bedeutungen besitzt,
mit einem Amin der Formel (III) umsetzt: 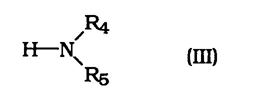 in der R₄ und R₅ die bezüglich der Formel (I) in Anspruch 1 angegebenen Bedeutungen besitzen, zur Bildung einer Verbindung der Formel (V): 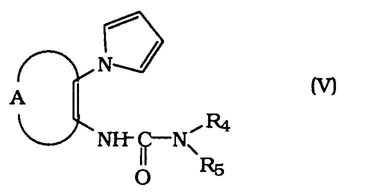 in der A, R₄ und R₅ die oben angegebenen Bedeutungen besitzen,
welche anschließend durch Einwirkung eines Halogenierungsmittels und Behandlung mit einem alkalischen Mittel cyclisiert wird zur Bildung einer Verbindung der Formel (I/c): 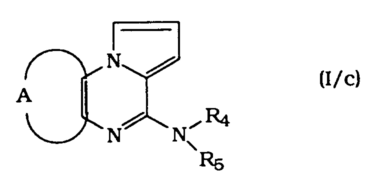 in der A, R₄ und R₅ die oben angegebenen Bedeutungen besitzen,
einem Sonderfall der Verbindungen der Formel (I) nach Anspruch 1, in der R₁ und R₂ gemeinsam eine Doppelbindung und R₃ eine Gruppe der Formel bedeuten: 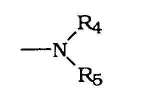 welche Verbindungen der Formel (I/c):
- nach einer oder mehreren Reinigungsmethoden ausgewählt aus Kristallisation, Säulenchromatographie über Siliciumdioxid, Extraktion, Filtration und Überführung über Kohlenstoff oder ein Harz gereinigt werden können,
- gegebenenfalls in ihre eventuellen optischen Isomeren in reiner Form oder in Form einer Mischung getrennt werden können,
- und/oder durch Einwirkung einer pharmazeutisch annehmbaren Säure oder Base in die Salze überführt werden können.

9. Pharmazeutische Zubereitung enthaltend als Wirkstoff mindestens eine Verbindung der Formel (I) nach Anspruch 1 oder eines ihrer Additionssalze mit einer pharmazeutisch annehmbaren Säure oder Base in Kombination mit einem oder mehreren pharmazeutisch annehmbaren Trägermaterialien oder Bindemitteln.

10. Pharmazeutische Zubereitung nach Anspruch 9 zur Vorbeugung und der Behandlung der Angst, der Depression, des Stresses, von Psychosen, der Schizophrenie, von Störungen des Zentralnervensystems, der Migräne, von Gedächtnisstörungen, von Ernährungsverhaltensstörungen, des Alkoholismus, von Schmerzen sowie zur Vorbeugung und Behandlung des Erbrechens und Störungen der Magenentleerung.
